# EUROPÄISCHE PATENTSCHRIFT

(11) **EP 1 881 853 B1**
(45) Veröffentlichungstag und Bekanntmachung des Hinweises auf die Patenterteilung: **28.09.2016**
(21) Anmeldenummer: 06742306.1
(22) Anmeldetag: 03.05.2006
(51) Int. Cl.: A61L 27/34, A61L 28/00, A61L 29/08, A61L 31/10

(54) **VOLLFLÄCHIGE BESCHICHTUNG VON GEFÄSSSTÜTZEN**
ALL-OVER COATING OF VESSEL STENTS
REVETEMENT DE TUTEURS VASCULAIRES, SUR TOUTE LEUR SURFACE

(30) Priorität: 05.05.2005 DE 102005021622; 06.06.2005 US 687340 P
(43) Veröffentlichungstag der Anmeldung: 30.01.2008
(73) Patentinhaber: Hemoteq AG, 52146 Würselen (DE)
(72) Erfinder: HORRES, Roland, 52223 Stolberg (DE); HOFFMANN, Michael, 52249 Eschweiler (DE); HOFFMANN, Erika, 52249 Eschweiler (DE); LINSSEN, Marita, 52066 Aachen (DE); CASPERS, Roger, 52459 Inden (DE); STYRNIK, Michaela, 52249 Eschweiler (DE)
(74) Vertreter: Arth, Hans-Lothar
(86) Internationale Anmeldenummer: PCT/DE2006/000766
(87) Internationale Veröffentlichungsnummer: WO 2006/116989

(56) Entgegenhaltungen:
- WO-A-02/24249
- WO-A-2005/030086
- WO-A-2005/032611

## Beschreibung

Die vorliegende Erfindung betrifft Verfahren zur vollflächigen Beschichtung von gitterartigen oder netzartigen Gefäßstützen, wobei die Gefäßstütze zuerst mit einer dünnen die materielle Oberfläche der Gefäßstütze, d.h. die das Gitter oder Netz bildenden Streben bedeckenden Schicht versehen werden und in einem zweiten Beschichtungsschritt eine vollflächige bzw. durchgängige Beschichtung der Gefäßstütze erfolgt, wobei diese vollflächige bzw. durchgängige Beschichtung die Streben als auch die Zwischenräume zwischen den einzelnen Streben überdeckt.

Krankhafte Veränderungen in und an allen Körperdurchgängen können zu Verengungen und gar völligem Verschluss führen. Neben der akuten Thrombose ist die Arteriosklerose, häufig Ursache für Herzinfarkt bzw. Schlaganfall.

Eine weitere und gleichfalls häufig auftretende Gefahr, die Körperpassagen trifft, ist das Wachstum von malignen und benignen Tumoren. Durch schnelle und unkontrollierte Zellteilung kommt es zur Ausbreitung des Tumors an und in den Hohlorganen und so zur Behinderung bzw. Verschlüssen in den Körperwegen. Als Beispiele seien der Speiseröhrenkrebs, der Darmkrebs, der Lungenkrebs, der Nierenkrebs, Verschlüsse der Gallenwege, der Bauchspeicheldrüse und der Harnröhre erwähnt.

Zur Behandlung verengter Blutgefäße hat sich in den letzten zwei Jahrzehnten der Stent als geeignete lokal wirkende Therapie bewährt. Er wird nach Weitung der betroffenen Stelle mit einem Ballonkatheter oder sogar Entfernung der Verengung an der betroffene Stelle angebracht, wo er in expandierter Form die Gefäßwand so nach außen drückt, dass der native Gefäßdurchmesser des betroffenen Gefäßes wiederhergestellt wird und dieses offen halten soll.

Besonders im Falle der mit Blut in Kontakt kommenden Stents verursachen diese als körperfremdes Material die Bildung von Restenosen. Bemühungen in der Weiterentwicklungen der Stents hin zu einer verbesserten Biokompatibilität des Stent-Materials, höheren Flexibilität bei geringer werdender Materialermüdung und Verkleinerung der Fremdoberfläche sollen das Risiko der Stent-induzierten Restenoserate immer weiter minimieren.

Als viel versprechende Weiterentwicklung hat sich bisher neben den genannten Grundbedingungen an den Stentkörper die Beschichtung der Stentoberfläche mit biokompatiblen bioabbaubaren oder biostabilen Materialien erwiesen, welche als Träger eines antirestenotisch agierenden Wirkstoffes fungieren. Dieser soll den restenosefördernden Prozess durch einen an die Erfordernisse zeitlich- und konzentrationsadaptierten Release stoppen. Dabei sind die Anforderungen nicht nur an den Stent selbst, sondern auch an das Beschichtungsmaterial, die Qualität der Beschichtung und an die Wirkstoffe gleichermaßen hoch.

Der gleiche Grundkörper wird genutzt, um beispielsweise Verengungen aufgrund von Tumorwachstum in der Speiseröhre oder in der Trachea bronchiale zu verhindern bzw. zu behindern. Diese Stents werden im Gegensatz zum die Arteriosklerose bekämpfenden vaskulären Stent mit einem den Stentkörper einhüllenden polymeren Mantel versehen, der als mechanische Barriere das erneute Einwachsen des Tumors durch die Zwischenräume in das Lumen verhindern oder wenigstens verlangsamen soll.

Allen in Körperhohlräumen eingesetzten Fremdmaterialien gemeinsam ist die Gewährleistung der möglichst uneingeschränkten Flexibilität also der physiologisch notwendigen ungestörten Beweglichkeit des Zielorgans bei gleichzeitiger Entfernung, oder Retardierung eingetretener lokaler Störungen des herkömmlich normalen Durchgangszustandes. Diese Flexibilität wird vom Material und Design des Hohlkörpers bestimmt und hat zu einem Hohlkörper mit geringer relativer Gefäßwandabdeckung und einer weitmaschigen bzw. netzartigen Struktur geführt.

Je nach Krankheitsbild und Einsatzort sind unterschiedliche Anforderungen an die Eigenschaften des Implantat zu beachten. So ergeben sich für eine Gefäßstütze, die in eine Arterie eingesetzt wird andere Voraussetzungen als beispielsweise für eine Gefäßstütze, die in die Speiseröhre eingesetzt wird. Doch während der vaskuläre beschichtete als auch unbeschichtete Stent möglichst wenig Fremdoberfläche aufweisen sollte, um die stentinduzierte Restenose zu verhindern, kann ein Stent in der Tumorbehandlung nur dann eine Barriere darstellen, wenn er den betroffenen Bereich vollständig abdecken kann. Dies ist nur dann möglich, wenn die stenttypischen grossen Zwischenräume nicht durchgängig bleiben und das Tumorwachstum zurückhalten können. Dies wird mit einer die Gefäßstütze umgebende Polymerhülle erreicht.

Doch während der vaskuläre Stent eine hämokompatible Oberfläche haben muss, wird beispielsweise für einen Ösophagusstent vor allem zwingend notwendig, dass der Stent trotz Hülle so fest sitzt, dass er nicht durch die Peristaltik des Schluckvorgangs in den Magen rutscht. Material der Gefäßstütze und Polymerhülle sollten zudem gegen Magensäure (Reflux, Erbrechen) unempfindlich sein.

Der Stent, der in der Trachea platziert werden soll, darf natürlich nicht die Atmung behindern und die Polymerhülle den Abfluss von Schleim und Sekret nicht stören. Er muss ebenfalls beim Niesen oder gar Husten, den dabei entstehenden hohen Drücken und Luftgeschwindigkeiten standhalten. Für die Nierengänge, den Harnleiter oder beispielsweise den Gallengängen gelten wiederum andere, dieser Umgebung angepasste, Voraussetzungen.

Dabei muss je nach Einsatzort gegebenenfalls auch zwischen der Oberflächenbeschaffenheit der Außenseite und der dem Lumen zugewandten Innenseite eines solchen Medizinproduktes differenziert werden.

Da der in Polymer eingehüllte Stent seine dem Standort angepasste Funktion sicher erfüllen soll und im Idealfall die ungestörte Funktion des Zielorgans gewährleisten bzw. unterstützen doch nicht negativ beeinflussen oder gar stören darf, wurden in der Vergangenheit verschiedene Konzepte ausgearbeitet, mit deren Hilfe ein Stent mit einer Polymerhülle versehen werden soll.

Doch ist die Anwendung von Stents für den nicht vaskulären Bereich noch nicht etabliert.

So beschreibt US5876448 (WO 93/22986) einen selbstexpandierenden ÖsophagusStent, dem im mittleren Bereich einen Silikonschlauch übergestülpt wird und diesen Bereich derart komprimiert, dass der Stent einen geringeren Durchmesser aufweist als in den schlauchfreien proximalen und distalen Endbereichen. Das proximale und distale Ende sind nicht eingehüllt, damit mit Hilfe dieser freien Stentstreben eine bessere Fixierung an den Wänden des Hohlraumes möglich wird. Doch hat sich dieser Stent nicht als erfolgreich erwiesen, da sich durch die Verengung des Stentkörpers Probleme ergeben, z.B. sind beim Erbrechen die Kräfte auf den Stent derart verstärkt, dass der Stent bewegt wird und mit den freien Stentenden die Speiseröhrenwand verletzt.

Zudem kann unter diesen Umständen der Silikonschlauch einreissen bzw. sich ablösen, zwischen Gefäßwand und Silikonbeschichtung kann sich Schleim oder Nahrungspartikel festsetzen, die neben der möglichen Entzündungsgefahr verschiedene für den Patienten äußerst negative Szenarien darstellbar machen.

WO 2005/030086 beschreibt eine Methode zur vollflächigen Beschichtung von ebenfalls selbstexpandierenden Stentkörpern, mit einer Polyurethanhülle, bei der nach einem ersten Spraycoating des Stents mit dem Polymer, das Polymer mit Hilfe eines Ballons oder einer anderen passenden hohlförmigen Schablone als Folie von innen an die Struts angelegt wird. Dabei erfolgt die den gesamten Stent überziehende Beschichtung von der Lumenseite, damit auf der Außenseite die Stentstreben weiterhin dem Stent Halt in der Gefäßwand geben können. Die anschließende Erhitzung des Systems über die Erweichungstemperatur soll das Polyurethan an den Stent binden. Hier gibt es Probleme, da die Polymerhülle nicht quantitativ an den beschichteten Stent gebunden wird und deshalb unter den gegebenen Bedingungen nicht dauerhaft auf dem Stent verbleibt. Ebenso können durch die Erhitzung kleine Löcher entstehen, die sich im Implantationsfall eventuell vergrössern und letztendlich zur Abtrennung von Beschichtungsmaterial und sogar zur Delokalisierung des gesamten Stent führen können.

Desweiteren kann das Erhitzen über den Erweichungspunkt des Polymeren dazu führen, dass einerseits das Spraycoating auf der Außenseite der Stent-Struts verläuft und andererseits die Haftung des Polymerüberzuges nicht nur an den Stent, sondern auch an den ebenfalls aus einem Polymer bestehenden Ballon erfolgt. Damit wird beim Zurückziehen des Ballons das innen liegende Coating Haftungsprobleme, das sich beim Entfernen des Ballons vom Stent zumindest teilweise ablöst. Dadurch kann sich zwischen sich lösendem Überzug und Innenwand Nahrung bzw. Schleim absetzen, der nach und nach die Beschichtung vom Stent trennt, aber vor allem den ungestörten Durchgang behindert. Das sich lösende Material ragt in das Gefäß hinein und führt zusätzlich zu Irritationen, Brechreiz oder Husten, was die Defixierung des gesamten Stentes fördert.

Besonders die Stentenden stellen Problembereiche für die Beschichtung dar, da oftmals in diesem Bereich die ersten Löcher in der Beschichtung entstehen, weil die Enden der Stentstreben sich bei Bewegung durch die sie bedeckende Schicht bohren. Dabei ist neben der damit möglichen Gefäßwandverletzung auch die weitere Lösung der Beschichtung erleichtert.

Ein Beispiel für einen partiell vollflächig beschichteten Stent ist in US5951599 zu finden. Der hierin beschriebene Stent soll zur Behandlung von Aneurysmen in Blutgefässen eingesetzt werden. Aneurysmen entstehen durch krankhafte Aussackung der Gefäßwand, in der sich unter immer größer werdenden Ausdehnung das Blut sammelt und gerinnt. Dies führt neben der wachsenden Thrombosegefahr letztendlich zur Gefäßruptur. US5951599 versucht dieses Problem zu lösen, indem die freien Zwischenräume eines vaskulären Stents mit einem kleinmaschigen Polymer-Netzwerk ausgefüllt werden, das im Blutgefäss über der Aussackung zu liegen kommen und das Aneurysma so abdeckt, dass der Blutfluss in der Aussackung stagniert. In Folge der fehlenden Bewegung bildet sich darin ein fester Thrombus, womit die Vergrößerung des Aneurysma gestoppt wird. Zudem soll die polymere Abdeckung verhindern, dass der Blutpfropf oder Teile des Gerinnsels in den Blutkreislauf gespült werden und an anderer Stelle einen Infarkt herbeiführen können. Auch hier kommt es aufgrund schlechter Haftung zu entsprechenden Problemen, die den Stent seiner Funktion berauben und so zu einem erhöhten Risiko für den Patienten führen. Zur Zeit werden Aneurysmen noch immer durch Ausfüllen mit Metalldraht ("Coils") behandelt, die so den Blutfluss innerhalb der Aussackung zum Erliegen bringen sollen.

Aufgabe der vorliegenden Erfindung ist es nun, ein Implantat bereitzustellen, welches die beschriebenen Nachteile vermeidet und ein optimales Herstellungsverfahren für derartige Implantate zur Verfügung zu stellen.

Diese Aufgabe wird durch die technische Lehre der unabhängigen Ansprüche der vorliegenden Erfindung gelöst. Weitere vorteilhafte Ausgestaltungen der Erfindung ergeben sich aus den abhängigen Ansprüchen, der Beschreibung sowie den Beispielen.

Es wurde gefunden, dass die Probleme des Standes der Technik dadurch gelöst werden können, dass man auf die Streben der Gefäßstütze eine erste untere Schicht aufbringt, welche die Zwischenräume zwischen den Streben bzw. in der Oberfläche der Gefäßstütze nicht überdeckt und danach die Schicht aufträgt, welche die Zwischenräume in der Oberfläche bzw. zwischen den einzelnen Streben der Gefäßstütze überdeckt und somit aus der gitterförmigen bzw. netzförmigen Struktur der Gefäßstütze eine zumindest teilweise vollflächige röhrenförmige Struktur erzeugt, wobei an den Kontaktstellen der beiden Polymerschichten rund um die einzelnen Streben der Gefäßstütze die beiden Schichten zu einer nicht mehr voneinander getrennten Einheit werden.

Eingesetzt für die Beschichtung werden somit Stützen für Körperdurchgänge, welche hierin auch allgemein als "Gefäße" bezeichnet werden, wie beispielsweise Adern, Venen, Speiseröhre, Gallengänge, Nierengänge, Luftröhre, Kanäle in den Bronchien, Dünndarmsegmente, Dickdarm oder andere annähernd rohrförmige Körperdurchgänge, wobei diese Gefäßstützen eine gitterförmige oder netzförmige Struktur aufweisen wie beispielsweise ein Stent. Der Begriff "Körperdurchgänge" umfasst dabei nicht nur natürliche Körperdurchgänge oder Körperkanäle, sondern auch künstliche Körperöffnungen und Körperkanäle wie beispielsweise Bypässe und künstliche Darmausgänge. Weitere Anwendungen für erfindungsgemäß beschichtete Gefäßstützen sind daher beispielsweise Kehlkopfimplantate, Bypässe, Katheter oder künstliche Darmausgänge und im Allgemeinen alle Bereiche im oder am lebenden Organismus, wo der Körperdurchgang frei als auch beweglich bleiben muss, wobei die Gefäßwände weiter möglichst optimal versorgt werden sollten, aber die Einwirkung des implantierten Fremdkörpers auf die Umgebung auf ein Minimum zu reduzieren ist.

Eine Gefäßstütze wie z.B. ein Stent bildet kein massives Rohr, sondern ein Gittergeflecht. Betrachtet man beispielsweise einen Stent, so wird dieser aus einem massiven Rohr z.B. mittels Laser geschnitten, so dass sich einzelne Streben ergeben, welche untereinander verbunden sind. Unter dem Begriff "Streben" wie hierin verwendet, sollen die einzelnen massiven Segmente (stent struts) des Stentgerüstes verstanden werden, welche an Knotenpunkten miteinander verbunden sind und die expandierbare und flexible Struktur der Gefäßstütze ausbilden.

Beim Schneiden eines Stent werden Segmente zwischen den einzelnen Streben herausgeschnitten, welche hierin als "Zwischenräume" bezeichnet werden. Eine Gefäßstütze weist daher eine Vielzahl von massiven Gerüstkomponenten (z.B. Streben, Ringen, Spiralen, Drähten und Knotenpunkte) auf, welche insgesamt die Gefäßstütze bilden sowie eine Vielzahl von Zwischenräumen zwischen diesen massiven Komponenten wie z.B. Knotenpunkten und Streben. Bei der gängigen Ausführungsform von Gefäßstützen laufen die Streben in Knotenpunkten zusammen, so dass die Zwischenräume durch die umgebenden Streben und Knotenpunkte definiert werden. Es gibt jedoch auch Ausführungsformen von Gefäßstützen, bei denen keine oder fast keine Knotenpunkte vorhanden sind und die Streben z.B. die Form von Ringen oder Spiralen haben. Bei solchen Gefäßstützen gibt es beispielsweise teilweise auch keine Vielzahl von Zwischenräumen mehr sondern nur noch wenige oder nur noch einen Zwischenraum, der durch beispielsweise zwei ineinanderlaufende Spiralen bestimmt wird. Derartige Zwischenräume sind dann zum Teil auch nicht mehr vollständig umgrenzt, sondern können ein oder zwei oder auch mehrere offene Enden oder offene Seiten aufweisen. Wie auch immer wird hierin unter "Zwischenräumen" der offene oder umgrenzte Bereich zwischen den massiven Komponenten der Gefäßstütze verstanden.

Die vorliegende Erfindung offenbart Beschichtungsverfahren, welche es zum einen ermöglichen, die Oberfläche der Gefäßstütze, welche zur Freisetzung eines pharmakologischen Wirkstoffs eingesetzt werden kann, zu vergrößern, um mehr Wirkstoff einbringen und gleichmäßig über eine größere Gefäßoberfläche applizieren zu können, ohne auf die einzelnen Streben eine sehr dicke Beschichtung auftragen zu müssen. Ferner ermöglichen die erfindungsgemäßen Beschichtungsverfahren für bestimmte Anwendungen wie beispielsweise die Offenhaltung von Körperdurchgängen, welche drohen von Krebsgeschwüren verschlossen zu werden, vollflächig beschichtete Gefäßstützen bereit zu stellen, welche das Gefäß dauerhaft offen halten und dennoch für eine Expansion flexibel genug sind.

Dies wurde dadurch erreicht, dass das gesamte gitterförmige bzw. netzartige Gerüst der Gefäßstütze vollflächig überzogen wird. Unter vollflächiger Beschichtung wird eine die Zwischenräume vollflächig überdeckende Beschichtung verstanden. Diese Beschichtung wird auch als durchgängig bezeichnet, d.h. es wird ein Film über einen Zwischenraum ausgebildet, der nur auf den diesen Zwischenraum umschließenden Streben aufliegt. Diese Beschichtung überspannt den Zwischenraum wie eine Hängebrücke, welche nur an den Rändern befestigt ist und im Zwischenraum auf keinem massiven Untergrund aufliegt. Damit diese vollflächige Beschichtung genügend an den Streben bzw. auf der Gefäßstütze haftet, werden in einem ersten Beschichtungsschritt mit einem Polymer A die Streben zumindest teilweise beschichtet, jedoch ohne die Zwischenräume zu überdecken und nach Benetzung bzw. Anlösung dieser ersten polymeren Beschichtung erfolgt dann die flächendeckende Beschichtung mit einem Polymer B in einem zweiten Beschichtungsschritt, wobei die erste polymere Beschichtung für eine bessere Anhaftung der zweiten vollflächigen bzw. durchgängigen polymeren Schicht sorgt. Dabei können auch Polymer A und Polymer B identisch sein und sich vorzugsweise nur in ihrer Konzentration in der Beschichtungslösung unterscheiden.

Die vorliegende Erfindung betrifft daher Verfahren gemäß Anspruch 1 zur vollflächigen Beschichtung von gitterartigen oder netzartigen Gefäßstützen insbesondere Stents, wobei in einem ersten Beschichtungsschritt die, die gitterartige oder netzartige Struktur ausbildenden Streben der Gefäßstütze vollständig oder teilweise mit einer polymeren Beschichtung überzogen werden und in einem zweiten Beschichtungsschritt die Zwischenräume zwischen den die gitterartige oder netzartige Struktur ausbildenden Streben mit einer polymeren Beschichtung vollflächig beschichtet werden.

Dies bedeutet, dass zuerst das massive Gerüst der Gefäßstütze mit einer polymeren Beschichtung versehen wird, wobei die Beschichtung um die einzelnen Streben liegt. Vorzugsweise werden mindestens 50%, weiter bevorzugt mindestens 70%, weiter bevorzugt mindestens 80%, noch weiter bevorzugt mindestens 90% und insbesondere bevorzugt mindestens 96% der Strebensegmente zwischen zwei Knotenpunkten mit der ersten polymeren Beschichtung versehen. Zudem ist bevorzugt auch mindestens 50%, weiter bevorzugt mindestens 70%, weiter bevorzugt mindestens 80%, noch weiter bevorzugt mindestens 90% und insbesondere bevorzugt mindestens 96% der Knotenpunkte mit der ersten Polymerschicht zu bedecken. Als Knotenpunkte werden solche Stellen bezeichnet, wo mindestens zwei Streben zusammenlaufen.

Die Streben bzw. Knotenpunkte werden mit der ersten Beschichtung wie ein Schlauch oder eine Isolierung um einen Draht umgeben, wobei diese Beschichtung jedoch nur die einzelnen Streben umgibt und nicht schon zwei benachbarte Streben miteinander verbindet. Die erste Beschichtung dient als Untergrund für eine bessere Anhaftung der nachfolgenden die Zwischenräume zwischen den Streben und Knotenpunkten überspannende Beschichtung.

Erfindungsgemäß ist es bevorzugt, die erste Beschichtung zu trocknen bzw. trocknen zu lassen bzw. aushärten zu lassen und danach wieder zu benetzen oder zu quellen oder anzulösen oder teilweise aufzulösen bevor die zweite Beschichtung aufgebracht wird. Die zweite Beschichtung überspannt bzw. überdeckte bzw. überzieht die Zwischenräume zwischen den Streben und Knotenpunkten des Gittergerüstes der Gefäßstütze zumindest teilweise. Teilweise bedeutet, dass mindestens 50%, weiter bevorzugt mindestens 70%, weiter bevorzugt mindestens 80%, noch weiter bevorzugt mindestens 90% und insbesondere bevorzugt mindestens 96% der Zwischenräume durchgängig bzw. vollflächig beschichtet sind. Die zweite die Zwischenräume bedeckende Polymerschicht bedeckt natürlich auch die Streben und Knotenpunkte, jedoch nicht zwangsläufig auch von deren innerer und äußerer Oberfläche. Aufgrund der unteren haftvermittelnden Polymerschicht ist es ausreichend, wenn beispielsweise die zweite Polymerschicht nur die äußere oder nur die innere Oberfläche der Streben und Knotenpunkte, d.h. der Gefäßstütze überdeckt.

Die einzelnen Streben oder Knotenpunkte der Gefäßstütze können zudem Aussparungen oder Kavitäten aufweisen, welche sich beispielsweise mit einem pharmakologischen Wirkstoff füllen und mit der ersten polymeren Beschichtung als auch mit der zweiten Beschichtung überdecken ließen. Eine derartige Überdeckung solcher Aussparungen und Kavitäten ist Stand der Technik und nur eine bevorzugte Ausführungsform aber nicht der wesentliche Aspekt der vorliegenden Erfindung.

Die unbeschichtete Gefäßstütze bzw. der Bare-Stent kann aus gängigen Materialien wie beispielsweise medizinischem Edelstahl, Titan, Chrom, Vanadium, Wolfram, Molybdän, Gold, Nitinol, Magnesium, Zink, Legierungen der vorgenannten Metalle oder Keramiken oder Polymeren bestehen. Diese Materialien sind entweder selbstexandierbar oder ballonexpandierbar und biostabil oder biologisch abbaubar. Das erfindungsgemäße Beschichtungsverfahren zur vollflächigen Beschichtung von gitterartigen oder netzartigen Gefäßstützen umfasst die folgenden Schritte:
a) Bereitstellung einer nicht vollflächigen, gitterartigen oder netzartigen Gefäßstütze mit Zwischenräumen zwischen den das Gitter oder Netz ausbildenden Streben,
b) zumindest teilweise Beschichtung der das Gitter oder Netz ausbildenden Streben mit einem Polymer A,
c) Benetzung der Oberfläche der mit dem Polymer A beschichteten Gefäßstütze mit einem organischen Lösungsmittel,
d) vollflächige Beschichtung der Zwischenräume zwischen den die gitterartige oder netzartige Struktur ausbildenden Streben mit einer polymeren Beschichtung eines Polymers B, wobei die vollflächige Beschichtung mit Polymer B über die Länge der Gefäßstütze hinausgeht und in einem weiteren Schritt um die Ränder der Gefäßstütze zur Außenseite gelegt und die entstehenden Kanten unter Druck und erhöhter Temperatur in die darunterliegende Schicht des Polymers B integriert wird.

Die Begriffe "nicht vollflächigen, gitterartigen oder netzartigen Gefäßstütze" bedeuten, dass die Gefäßstütze nicht die Form eines massiven Rohres oder Zylinders hat. "Zumindest teilweise" oder "zumindest eines Teils" wurde bereits oben durch die Prozentangaben definiert und der Begriff "Benetzung" umfasst nicht nur ein Anfeuchten der ersten Polymerschicht sondern auch ein Quellen, Anlösen oder teilweises Auflösen der ersten Schicht. Die Benetzung erfolgt vorzugsweise durch Besprühen der Gefäßstütze mit einem organischen Lösungsmittel oder Lösungsmittelgemisch oder Eintauchen der Gefäßstütze in ein organisches Lösungsmittel oder Lösungsmittelgemisch oder auch nur Aufbewahrung der Gefäßstütze in einer mit einem organischen Lösungsmittel oder Lösungsmittelgemisch gesättigten Atmosphäre.

Eine weitere Ausführungsform der vorliegenden Erfindung ist ein Verfahren zur vollflächigen Beschichtung von gitterartigen oder netzartigen Gefäßstützen umfassend die folgenden Schritte:
a) Bereitstellung einer nicht vollflächigen, gitterartigen oder netzartigen Gefäßstütze umfassend Streben mit einer inneren Oberfläche und einer äußeren Oberfläche mit Zwischenräumen zwischen den einzelnen Streben,
b) zumindest teilweise Beschichtung der inneren als auch der äußeren Oberfläche der Streben mit einem Polymer A,
c) Benetzung der Oberfläche der mit dem Polymer A beschichteten Gefäßstütze mit einem organischen Lösungsmittel,
d) vollflächige Beschichtung der inneren und/oder äußeren Oberfläche sowie der Zwischenräume zwischen den Streben mit einer polymeren Beschichtung eines Polymers B, wobei die vollflächige Beschichtung mit Polymer B über die Länge der Gefäßstütze hinausgeht und in einem weiteren Schritt um die Ränder der Gefäßstütze zur Außenseite gelegt und die entstehenden Kanten unter Druck und erhöhter Temperatur in die darunterliegende Schicht des Polymers B integriert wird.

Geht man von annähernd runden Streben aus, so wird als "innere Oberfläche" der eine Teil der Oberfläche der Streben bezeichnet, der zur Längsachse der Gefäßstütze gerichtet ist und als "äußere Oberfläche" der zur inneren Oberfläche gegenüberliegende Teil. Es wird daher nur zwischen äußerer und innerer Oberfläche differenziert.

Vorzugsweise wird der Beschichtungsschritt b) mittels Sprühverfahren oder electro spinning ausgeführt, während die Schritte c) und d) bevorzugt mittels Tauchverfahren, Mikropipettiertechnik, Electrospinning, oder/und "Seifenblasenverfahren" durchgeführt werden.

Die polymere Oberfläche kann in einem weiteren Schritt mit einem Polymer C vollständig oder teilweise von innen und/oder außen beschichtet werden.

So ist beispielsweise für die luminale Seite eines Trachea Bronchial-Stents von Wichtigkeit, dass diese gleitfähig genug ist, damit der Abtransport von Sekret, Schleim etc. nicht behindert wird. Die Hydrophilie kann durch Beschichtung mit einem geeigneten Polymer wie beispielsweise Polyvinylpyrrolidon (PVP) gesteigert werden.

Die verwendeten vorzugsweise unbeschichteten Gefäßstützen haben eine Struktur, welche nicht vollflächig ist, sondern Aussparungen oder Zwischenräume zwischen innerer und äußerer Oberfläche aufweisen.

Der Bezug auf innere und äußere Oberflächen lässt erkennen, dass die Gefäßstützen, welche bei dem erfindungsgemäßen Verfahren verwendet werden können, vorzugsweise eine röhrenförmige Struktur besitzen. Die zu beschichtenden Gefäßstützen sind vorzugsweise länglich und innen hohl, so dass röhrenförmige, spiralförmige, zylinderförmige, netzartige, geflochtene und/oder gitterartige Strukturen bevorzugt sind.

Mit diesem Beschichtungsverfahren lassen sich zum jetzigen Stand der Technik auftretenden und erwähnten Nachteile einer flächendeckenden Beschichtung verhindern und die damit für den Patienten entstehenden Risiken vermeiden. Derartige erfindungsgemäß einsetzbare Medizinprodukte können einerseits beschichtet werden, indem auf dem massiven Material, beispielsweise den einzelnen Streben eines Stents eine Beschichtung aufgetragen wird und die die durch die Streben begrenzte freie Ebene mit einer polymeren Schicht B gefüllt wird. Diese polymere Schicht vermag aufgrund der Polymereigenschaften die Zwischenräume der mit Polymer A beschichteten Stentstreben zu überdecken. Die Stabilität der Einhüllung wird durch den Verbund der beiden Schichten des Polymeren B mit dem Polymeren A rund um die Elemente des Medizinproduktes bestimmt.
Somit können alle Medizinprodukte erfindungsgemäß beschichtet werden, welche eben solche Zwischenräume in der Oberflächenstruktur aufweisen, wie es beispielsweise Stents zwischen den einzelnen Stentstreben tun.

Die Beschichtung der massiven Teile, beispielsweise der einzelnen Streben von Gefäßstützen, erfolgt vorzugsweise mittels Sprühverfahren oder electro spinning und die erhaltene Schicht ist deutlich dünner als die vollflächige Beschichtung. Der erste Beschichtungsschritt kann aber auch im Tauchverfahren, Plasmaabscheidungsverfahren oder Gasphasenbeschichtungsverfahren durchgeführt werden.

Bei der ersten Beschichtung wird vorzugsweise ein Polymer A und beim zweiten Beschichtungsschritt ein Polymer B eingesetzt, wobei es auch möglich ist, bei beiden Beschichtungsschritten dasselbe Polymer zu verwenden.

Dabei ist die Verwendung eines bioabbaubaren oder/und biostabilen Polymers A für die erste Beschichtung und eines biologisch abbaubaren oder resorbierbaren Polymer B oder/und biostabiles Polymer für die überdeckende zweite Beschichtung in Abhängigkeit von der Art der Anwendung möglich.

Als biologisch stabile und nur langsam biologisch abbaubare Polymere können genannt werden: Polyacrylsäure und Polyacrylate wie Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyvinylaromaten, Polyvinylester, Polyvinylpyrollidone, Polyoxymethylene, Polyethylen, Polypropylen, Polytetrafluorethylen, Polyurethane, Polyolefin-Elastomere, Polyisobutylene, EPDM-Gummis, Fluorosilicone, Carboxymethylchitosan, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetat-butyrate, Ethylvinylacetat-copolymere, Polysulfone, Polyethersulfone, Epoxyharze, ABS-Harze, EPDM-Gummis, Siliconpräpolymere, Silicone wie Polysiloxane, Polyvinylhalogene und Copolymere, Celluloseether, Cellulosetriacetate, Chitosan, Chitosanderivate, polymerisierbare Öle wie z.B. Leinöl und Copolymere und/oder Mischungen derselben.

Ferner lässt sich in einem dem Beschichtungsschritt mit Polymer A vorgelagerten Schritt eine hämokompatible Schicht vorzugsweise kovalent auf die unbeschichtete Oberfläche des Medizinproduktes gebundene oder mittels Quervernetzung z.B. mit Glutardialdehyd auf der Oberfläche des Medizinproduktes immobilisieren. Eine solcherart die Blutgerinnung nicht aktivierende Schicht ist dann sinnvoll, wenn unbeschichtetes Stentmaterial in Kontakt mir Blut kommmen kann. So ist zu bevorzugen, einen partiell beschichteten Stent, wie er z.B. in US595159 zur Behandlung von Aneurysmen beschrieben wird, zunächst mit dieser hämokompatible Schicht auszustatten.

Die vorzugsweise hämokompatible Schicht wird aus folgenden bevorzugten Stoffen hergestellt: Heparin nativen Ursprungs als auch regioselektiv hergestellte Derivate unterschiedlicher Sulfatierungsgrade und Acetylierungsgrade im Molekulargewichtsbereich von des für die antithrombotische Wirkung verantwortlichen Pentasaccharides bis zum Standardmolekulargewicht des käuflichen Heparins von ca. 13kD, Heparansulfate und seine Derivate, Oligo- und Polysaccharide der Erythrocytenglycolcalix, Oligosaccharide, Polysaccharide, vollständig desulfatiertes und N-reacetyliertes Heparin, desulfatiertes und N-reacetyliertes Heparin, N-carboxymethyliertes und/oder partiell N-acetyliertes Chitosan, Polyacrylsäure, Polyetheretherketone, Polyvinylpyrrolidon, und/oder Polyethylenglykol sowie Mischungen dieser Substanzen.

Als biologisch abbaubare oder resorbierbare Polymere können beispielsweise verwendet werden: Polyvalerolactone, Poly-s-Decalactone, Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäureanhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere wie z.B. aus Oligocaprolactondiole und Oligodioxanondiole, Polyetherester-multiblockpolymere wie z.B. PEG und Poly(butylenterephtalat. Polypivotolactone, Polyglycolsäuretrimethyl-carbonate Polycaprolacton-glycolide, Poly(g-ethylglutamat), Poly(DTH-Iminocarbonat), Poly(DTE-co-DT-carbonat), Poly(Bisphenol A-iminocarbonat), Polyorthoester, Polyglycol-säuretrimethylcarbonate, Polytrimethylcarbonate, Polyiminocarbonate, Poly(N-vinyl)-Pyrolidon, Polyvinylalkohole, Polyesteramide, glycolierte Polyester, Polyphosphoester, Polyphosphazene, Poly[p-carboxyphenoxy)propan] Polyhydroxypentan-säure, Polyanhydride, Polyethylenoxid-propylenoxid, weiche Polyurethane, Polyurethane mit Aminosäurereste im Backbone, Polyetherester wie das Polyethylenoxid, Polyalkenoxalate, Polyorthoester sowie deren Copolymere, Carrageenane, Fibrinogen, Stärke, Kollagen, protein-basierte Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, desweiteren Hyaluronsäure, Heparansulfat, Heparin, Chondroitinsulfat, Dextran, b-Cyclodextrine, und Copolymere mit PEG und Polypropylenglycol, Gummi arabicum, Guar, Gelatine, Collagen Collagen-N-Hydroxysuccinimid, Modifikationen und Copolymere und/oder Mischungen der vorgenannten Substanzen.

Ferner ist bevorzugt, dass bei dem zweiten vollflächigen Beschichtungsschritt keine glatte oder plane äußere Oberfläche resultiert, sondern beispielsweise bei einem Stent die Stentstruktur, d.h. die Struktur der Streben erkenntlich bleibt. Dies hat den Vorteil, dass die äußere zur Gefäßwand gerichtete beschichtete Oberfläche der Gefäßstütze eine gewellte und unebene Struktur aufweist, wodurch ein besserer Halt im Gefäß garantiert wird.

Ferner ist bevorzugt, wenn der zweite Beschichtungsschritt in zwei Stufen abläuft. Die zu beschichtenden Medizinprodukte sind zumeist vorzugsweise röhrenförmig mit zumindest einem offenen Ende an einer Seite zumeinst jedoch an beiden Seiten. Zur Ausführung des zweiten Beschichtungsschritts [Schritte c), d)] wird dieses Medizinprodukt vorzugsweise auf einen Stab oder Stift gewünschten Durchmessers aufgesetzt und in ein geeignetes Lösungsmittel getaucht. Der Stab oder Stift hat den Durchmesser, der dem gewünschten späteren Innendurchmesser des Medizinproduktes nach erfolgter Beschichtung entsprechen soll.
Bevorzugt ist ferner, wenn das Medizinprodukt mit Hilfe des Tauchverfahren derart beschichtet wird, dass die, obwohl von außen aufgezogene, Schicht auf der Innenseite des Medizinproduktes eine glatte, gleichmäßige Beschichtung ergibt, so dass auf der Außenseite die Oberflächenstruktur des Medizinproduktes unter der Beschichtung hervortritt bzw. beibehalten werden kann. Um dies zu gewährleisten ist der erste Beschichtungsschritt notwendig, da erst durch die Beschichtung mit Polymer A sich zwischen dem zur vollflächigen Tauch-Beschichtung erforderlichen Stabes bzw. Stiftes und den Stentstreben ein der Polymerschicht entsprechender Abstand zwischen Stentstreben und Stab generieren. Die Polymerlösung B kann nun in diese von der luminalen Seite des Medizinproduktes offenen Zwischenbereiche fließen und diese auffüllen.

Als Lösungsmittel werden vorzugsweise solche gut benetzenden organischen Lösungsmittel verwendet, welche das Polymer B gut lösen. Das verwendete Lösungsmittel kann bereits das Polymer B in einer geringen Konzentration enthalten, so dass die Viskosität der Lösung noch derart gering ist, dass eine gute und vollständige Benetzung insbesondere der inneren Oberfläche des Medizinproduktes erfolgt. Die Konzentration des Polymeren B in dem zur Benetzung verwendeten Lösungsmittel gemäß Schritt c) ist geringer als die Konzentration des Polymeren B in der zur Beschichtung verwendeten Lösung gemäß Schritt d). Als Lösungsmittel werden Lösungsmittel mit geringem Dampfdruck bevorzugt wie Aceton, THF, Methylenchlorid, Chloroform, Tetrachlorkohlenstoff, Methanol, Ethanol, Ether, Petrolether, Hexan, Heptan, Cyclohexan, Benzol, Toluol, Essigsäureethylester oder Essigsäure eingesetzt.

Der Benetzungsschritt c) hat den Vorteil, dass dadurch der Einschluss von Luftblasen in der vollflächigen Polymerschicht aus Polymer B vermieden wird und eine intensive Anhaftung bzw. Verschmelzung der Polymerschicht aus Polymer A und der Polymerschicht aus Polymer B stattfindet, da beide Polymere im verwendeten Lösungsmittel entweder vollständig (A und B) oder teilweise (A) löslich sind.

Anstelle des Benetzungsschrittes oder zusätzlich zu dem Benetzungsschritt können die beiden Polymerschichten unter Einwirkung von Wärme miteinander verschmolzen werden.

Das mit Lösungsmittel benetzte oder bereits mit einer verdünnten Lösung des Polymeren B benetzte oder teilweise beschichtete Medizinprodukt wird dann mit einer konzentrierteren Lösung des Polymeren B vollflächig beschichtet. Dies kann geschehen, indem man das Medizinprodukt in eine konzentriertere Tauchlösung B überführt oder die verdünnte Lösung des Polymeren B aufkonzentriert, wobei die erste Ausführungsform bevorzugt ist.

Ferner ist bevorzugt, wenn die Benetzungslösung und die Beschichtungslösung dasselbe Lösungsmittel enthalten, wobei dies aber nicht zwingend der Fall sein muss. Wichtig ist jedoch, dass das Lösungsmittel für die Benetzungslösung und das Lösungsmittel für die vollflächige Beschichtung gemäß Schritt d) miteinander mischbar sind und vorzugsweise das Polymere B gleich gut lösen oder nicht zu einer Fällung des Polymeren B oder zu einer Trübung der Beschichtungslösung führen.

Als Spezialfall des Tauchverfahrens lässt sich zur vollflächigen Beschichtung des Medizinproduktes die Grenzflächenpolykondensation nutzen. Bei diesem Verfahren besteht die Tauchlösung aus zwei nicht miteinander mischbaren Monomerlösungen, wobei sich an der Phasengrenzfläche das in beiden Monomerlösungen nicht lösliche Polymer als dünner Film bildet. Mit dem vorsichtigen langsamen Herausziehen dieses Films als Faden oder Schlauch aus der Mitte des Reaktionsgefässes bildet sich im gleichen Masse vom Rande des Gefässes der Film nach. Dies geschieht so lange, bis die Monomerlösungen so verdünnt sind, dass der Schlauch reißt.
Bringt man nun den Stent in die untere Lösung, kann er bei geeigneter Hebegeschwindigkeit an der Grenzfläche den Polymerfilm heben, so dass dieser sich wie ein Schlauch um den zu beschichtenden Körper legt.
Dieses Verfahren eignet sich in eleganter Weise zur Ausbildung der vollflächigen Polymerbeschichtung aus beispielsweise einem Säurechlorid und Diaminen oder Diolen. Als Reaktionsgefäß dient ein U-förmiges Rohr. Das Säuredichlorid wird vorzugsweise in einem organischen Lösungsmittel mit einer Dichte größer 1 g/ml gelöst. Als organische Lösungsmittel oder Lösungsmittelgemische bieten sich Methylenchlorid, Chloroform oder Tetrachlorkohlenstoff an. Zu der organischen Lösung mit dem Säuredichlorid kann beispielsweise auch ein pharmakologischer Wirkstoff zugesetzt werden, sofern dieser nicht mit dem Säurechlorid reagiert. Das Diamin (oder z.B. Diol) wird in Wasser gelöst. Es ist möglich, dieser wäßrigen Lösung einen hydrophilen pharmakologischen Wirkstoff zuzusetzen, sofern dieser nicht mit dem Diamin bzw. Diol reagiert. Die organische Lösung des Säuredichlorids eventuell mit lipophilem pharmakologischem Wirkstoff wird in das U-förmige Reaktionsgefäß gefüllt und nur ein Schenkel des Reaktionsgefäßes wird mit der wäßrigen Lösung des Diamins eventuell mit hydrophilem pharmakologischem Wirkstoff derart gefüllt, dass nur in diesem Schenkel die Grenzphasenpolymerisation stattfindet, welche nach Ausbildung eines Grenzflächenfilms abbricht. Die mit der ersten Beschichtung versehenen Gefäßstützen werden auf einen Dorn aufgesetzt und tauchen entlang eines Führungsdrahtes in den Schenkel den Reaktionsgefäßes ein, der nur die organische Lösung des Säuredichlorids enthält und passieren den Tiefpunkt des Reaktionsgefäßes. Die Zeit für diese Bewegung reicht aus, um ein Quellen oder Anlösen oder teilweises Auflösen der bereits vorhandenen Polymerbeschichtung um die einzelnen Streben zu bewirken. Die Gefäßstützen werden in dem zweiten Schenkel des Reaktionsgefäßes herausgezogen, wo sich der polymere Grenzfilm ausgebildet hat und die organische Lösung mit der wäßrigen Lösung des Diamins überschichtet ist. Bei der Passierung der Grenzfläche haftet der Film an der Gefäßstützen und wird zusammen mit der Gefäßstütze herausgezogen, wobei sich der Film wie ein Schlauch nachbildet und sich beim Herausziehen wie ein Mantel um die Gefäßstütze legt.

Bei einer bevorzugten Ausführungsform wird die organische Lösung sowie die überstehende wäßrige Lösung computergesteuert nachgefüllt und die Gefäßstützen wandern wie auf einem Fließband hintereinander durch die Reaktionsapparatur, so dass der polymere Grenzfilm nicht abreißt oder durchtrennt werden muss und sich wie ein Schlauch um eine Vielzahl von hintereinandergereihten Gefäßstützen legt. Die Durchtrennung des Polymerschlauches das Einklappen bzw. Umklappen seiner Ränder um die Enden der Gefäßstütze zu deren Verstärkung sowie die eventuelle weitere Aushärtung der vollflächigen Polymerbeschichtung erfolgt je nach Anforderungen und Polymer nach bekannten Standardverfahren.

Die vollflächige Beschichtung eines Medizinproduktes gemäß Schritt d) kann auch mit Hilfe der Mikropipettierung der einzelner Zwischenräume vorgenommen werden. Dabei werden die ausgewählten Bereiche des Medizinproduktes mit der Mikropipette derart befüllt, dass der Polymerfilm die Freifläche zwischen den begrenzenden Elementen des Medizinproduktes gleichmässig überzieht.

Die Beschichtung in Schritt d) lässt sich auch mit dem sogenannten Electrospinning gestalten, wobei die Variation der Reaktionsparameter die Möglichkeit bieten, einzelne Polymerfäden mit Durchmessern im Nanobereich bis hin zu flächendeckenden Schichten herzustellen und gezielt auf ein Objekt aufzutragen. Dieses Verfahren bietet die Möglichkeit die freien Räume nach Bedarf auch strukturiert zu gestalten, wie es beispielsweise für die Anwendung des tissue engineering notwendig ist.

Eine weitere Variante zur vollflächigen Beschichtung derartiger Implantate besteht darin, Lösungen geeigneter Elastomere über einen Ring ähnlich der Seifenblasenbildung mit Stickstoff oder Sauerstoff oder einem anderen geeigneten Gas zu einem Ballon aufzu"pusten". In diesem Ballon lässt sich die Gefäßstütze mit einem genau mittig zum Ringdurchmesser platzierten Stift während der Blasenbildung problemlos einführen.

Bringt man den Hohlkörper in solch einen aus einer Polymerlösung gebildeten "Ballon", bevor dieser aufgrund des immer geringer werdenden Umfangs wieder kleiner wird, kann sich der Polymerfilm eng um den Hohlkörper legen und die Streben als Erhöhung sichtbar werden. In einem zweiten Schritt wird der außen beschichtete Körper von innen beschichtet werden, so dass zum einen innen eine glatte Oberfläche entsteht, und zum anderen das Polymer auf der äußeren Oberfläche mit dem inneren Polymeren zu einer Einheit werden. Dies lässt sich beispielsweise erreichen, indem man den beschichteten Stent auf ein Formwerkzeug bringt, dessen Durchmesser wenig geringer ist als die der beschichteten Gefäßstütze und füllt den Zwischenraum mit einer Lösung des Polymeren. Der derart von innen beschichtete Hohlkörper wird anschließend vorsichtig vom Werkzeug entfernt und getrocknet.
Die Grenzflächenspannung und Kohäsionskräfte einer Polymerlösung lassen sich gleichfalls zur partiellen Beschichtung nutzen, indem der Polymerfilm über einzelner Segmente gezogen oder geschoben werden kann.

Die Lösung für die erste Beschichtung enthält vorzugsweise eine Polymerkonzentration an Polymer A von 0,01 bis 80 Gew.-%, bevorzugt von 0,1 bis 50 Gew.-%, insbesondere bevorzugt von 0,5 bis 25 Gew.-%.

Die Schichtdicke dieser ersten unteren vorzugsweise im Sprühverfahren oder Electrospinning aufgebrachte Schicht liegt bevorzugt zwischen 0,0001 und 1000 µm, weiter bevorzugt zwischen 0,001 und 500µm und insbesondere bevorzugt zwischen 0,1 und 250 µm.

Die Konzentration an Polymer B für die vollflächige Beschichtung liegt vorzugsweise zwischen 0,1 und 80Gew.-%, weiter bevorzugt zwischen 1 und 50Gew.-% und insbesondere bevorzugt zwischen 5 und 30Gew.-%.

Die Schichtdicke der zweiten Polymerschicht aus dem Polymer B oder C optional enthaltend einen oder mehrere pharmakologische Wirkstoffe liegt vorzugsweise zwischen 0,1 und 1500 µm, weiter bevorzugt zwischen 1 und 1000 µm und insbesondere bevorzugt zwischen 2 und 500 µm.

Ferner ist bevorzugt, wenn die äußere Schicht auf der inneren Oberfläche des Medizinproduktes bzw. der Gefäßstütze aus einem anderen Polymeren B' besteht wie auf der äußeren Oberfläche, welche beispielsweise mit dem Polymer B" beschichtet ist. Derartige Beschichtungen lassen sich realisieren, indem beispielsweise das Medizinprodukt bzw. die Gefäßstütze auf einen Stift oder Stab aufgesetzt wird, der einen geringen Durchmesser als das Medizinprodukt bzw. die Gefäßstütze selbst hat und danach innere sowie äußere Oberfläche des Medizinproduktes bzw. der Gefäßstütze mit dem Polymer B' beschichtet werden und danach ein weiterer Beschichtungsschritt folgt, wo nur noch die äußere Oberfläche mit dem Polymer B" beschichtet werden kann, da der Zwischenraum zwischen innerer Oberfläche des Medizinproduktes bzw. der Gefäßstütze und Oberfläche des Stiftes oder Stabes bereits vollständig mit dem Polymer B' ausgefüllt ist. Eine andere Möglichkeit besteht darin, einen Stift oder Stab zu verwenden, der denselben Durchmesser wie das Medizinprodukt bzw. die Gefäßstütze hat und die äußere Oberfläche sowie die Zwischenräume mit einem Polymer B' beschichtet werden und danach im Inneren des Medizinproduktes bzw. der Gefäßstütze eine weitere Beschichtung angewendet wird, welche zur Aufbringung des Polymeren B" dient.

Die Aufbringung der ersten unteren und dünnen Beschichtung ist insbesondere vorteilhaft bei dem Einsatz von Medizinprodukten bzw. Gefäßstützen aus Metall, Metallsalzen, Metalllegierungen oder Mischungen der vorgenannten Materialien, um vor lonenaustritt, Korrosion sowie der Bildung von Lokalelementen zu schützen.

Bei weiteren bevorzugten Ausführungsformen werden Lösungen des Polymers A und/oder des Polymers B verwendet, welche ferner mindestens einen antiproliferativen, antimigrativen, antiangiogenen, antiinflammatorischen, antiphlogistischen, zytostatischen, zytotoxischen und/oder antithrombotischen Wirkstoff enthalten. Dieser Wirkstoff kann in kovalent gebundener Form oder in adhäsiv oder ionisch gebundener Form enthalten sein. Dadurch werden beschichtete Medizinprodukte bzw. Gefäßstützen erhalten, welche mindestens einen Wirkstoff in der Polymerschicht aus Polymer A und/oder vorzugsweise mindestens einen Wirkstoff in der Polymerschicht aus Polymer B enthalten, vorzugsweise in Form einer wirkstofffreisetzenden Schicht (drug release system). Natürlich ist es auch möglich, den oder die Wirkstoffe in einem weiteren Beschichtungsschritt auf die erste oder bevorzugt zweite Schicht aufzutragen, so dass eine weitere Wirkstoffschicht vorhanden ist.

Die Konzentration pro Wirkstoff liegt vorzugsweise im Bereich von 0,001-500 mg pro cm² vollflächig beschichteter Oberfläche der Gefäßstütze, d.h. die Oberfläche wird unter Berücksichtigung der Gesamtoberfläche der beschichteten Streben und der Oberfläche der überdeckten Zwischenräume zwischen den Streben berechnet.

Der oder die Wirkstoffe können sich je nach Beschichtungsverfahren unter, in und/oder auf der ersten und/oder zweiten Polymerschicht befinden. Als antiproliferative, antimigrative, antiangiogene, antiinflammatorische, antiphlogistische, zytostatische, zytotoxische und/oder antithrombotische Wirkstoffe können bevorzugt eingesetzt werden: Sirolimus (Rapamycin), Everolimus, Pimecrolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid, Estramustin, Melphalan, Ifosfamid, Tropfosfamid, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, 8-α-Ergoline, Dimethylergoline, Agroclavin, 1-Allylisurid, 1-Allyltergurid, Bromergurid, Bromocriptin (Ergotaman-3',6',18-trione, 2-bromo-12'-hydroxy-2'-(1-methylethyl)-5'-(2-methylpropyl)-, (5'alpha)-), Elymoclavin, Ergocristin (Ergotaman-3',6',18-trione, 12'-hydroxy-2'-(1-methylethyl)-5'-(phenyimethyl)-, (5'-alpha)-), Ergocristinin, Ergocornin (Ergotaman-3',5',18-trione, 12'-hydroxy-2',5'-bis(1-methylethyl)-, (5'-alpha)-), Ergocorninin, Ergocryptin (Ergotaman-3',6',18-trione, 12'-hydroxy-2'-(1-methylethyl)-5'-(2-methylpropyl)-, (5'alpha)- (9Cl)), Ergocryptinin, Ergometrin, Ergonovin (Ergobasin, INN: Ergometrin, (8beta(S))-9,10-Didehydro-N-(2-hydroxy-1-methylethyl)-6-methyl-ergoline-8-carboxamid), Ergosin, Ergosinin, Ergotmetrinin, Ergotamin (Ergotaman-3',6',18-trione, 12'-hydroxy-2'-methyl-5'-(phenylmethyl)-, (5'-alpha)- (9Cl)), Ergotaminin, Ergovalin (Ergotaman-3',6',18-trione, 12'-hydroxy-2'-methy)-5'-(1-methylethyl)-, (5'alpha)-), Lergotril, Lisurid (CAS-Nr.: 18016-80-3, 3-(9,10-Didehydro-6-methylergolin-8alpha-yl)-1,1-diethylharnstoff), Lysergol, Lysergsäure (D-Lysergsäure), Lysergsäureamid (LSA, D-Lysergsäureamid), Lysergsäurediethylamid (LSD, D-Lysergsäurediethylamid, INN: Lysergamid, (8beta)-9,10-Didehydro-N,N-diethyl-6-methyl-ergoline-8-carboxamid), Isolysergsäure (D-Isolysergsäure), Isolysergsäureamid (D-Isolysergsäureamid), Isolysergsäurediethylamid (D-Isolysergsäurediethylamid), Mesulergin, Metergolin, Methergin (INN: Methylergometrin, (8beta(S))-9,10-Didehydro-N-(1-(hydroxymethyl)propyl)-6-methyl-ergoline-8-carboxamid), Methylergometrin, Methysergid (INN: Methysergid, (8beta)-9,10-Didehyd ro-N-(1-(hydroxymethyl)propyl)-1,6-d imethyl-ergoline-8-carboxamid), Pergolid ((8beta)-8-((Methylthio)methyl)-6-propyl-ergolin), Protergurid und Tergurid, Celecoxip, Thalidomid, Fasudil^{®}, Ciclosporin, SMC-Proliferation-Inhibitor-2w, Epothilone A und B, Mitoxanthrone, Azathioprin, Mycophenolatmofetil, c-myc-Antisense, b-myc-Antisense, Betulinsäure, Camptothecin, PI-88 (sulfatiertes Oligosaccharid), Melanocyte-stimulating hormon (α-MSH), aktiviertes Protein C, IL1-ß-Inhibitor, Thymosin α-1, Fumarsäure und deren Ester, Calcipotriol, Tacalcitol, Lapachol, ß-Lapachon,Podophyllotoxin, Betulin, Podophyllsäure-2-ethylhydrazid, Molgramostim (rhuGM-CSF), Peginterferon α-2b, Lanograstim (r-HuG-CSF), Filgrastim, Macrogol, Dacarbazin, Basiliximab, Daclizumab, Selectin (Cytokinantagonist), CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, 1,11-Dimethoxycanthin-6-on, 1-Hydroxy-11-Methoxycanthin-6-on, Scopolectin, Colchicin, NO-Donoren wie Pentaerythrityltetranitrat und Syndnoeimine, S-Nitrosoderivate, Tamoxifen, Staurosporin, ß-Estradiol, α-Estradiol, Estriol, Estron, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A, Paclitaxel und dessen Derivate wie 6-α-Hydroxy-Paclitaxel, Baccatin, Taxotere, synthetisch hergestellte als auch aus nativen Quellen gewonnene macrocyclische Oligomere des Kohlensuboxids (MCS) und seine Derivate, Mofebutazon, Acemetacin, Diclofenac, Lonazolac, Dapson, o-Carbamoylphenoxyessigsäure, Lidocain, Ketoprofen, Mefenaminsäure, Piroxicam, Meloxicam, Chloroquinphosphat, Penicillamin, Tumstatin, Avastin, D-24851, SC-58125, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin, Tobramycin, Gentamycin, Penicilline wie Dicloxacillin, Oxacillin, Sulfonamide, Metronidazol, Antithrombotika wie Argatroban, Aspirin, Abciximab, synthetisches Antithrombin, Bivalirudin, Coumadin, Enoxoparin, desulfatiertens und N-reacetyliertes Heparin, Gewebe-Plasminogen-Aktivator, Gpllb/Illa-Plättchenmembranrezeptor, Faktor Xₐ-Inhibitor Antikörper, Interleukininhibitoren, Heparin, Hirudin, r-Hirudin, PPACK, Protamin, Natriumsalz der 2-Methylthiazolidin-2,4-dicarbonsäure Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon α, ß und γ, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol, Vitamin B1, B2, B6 und B12, Folsäure, Tranilast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, D24851, SC-58125, Retinolsäure, Quinidin, Disopyrimid, Flecainid, Propafenon, Sotolol, Amidoron, natürliche und synthetisch hergestellte Steroide wie Bryophyllin A, Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Antimykotika wie Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceanole A, B und C, Bruceantinoside C, Yadanzioside N und P, Isodeoxyelephantopin, Tomenphantopin A und B, Coronarin A, B, C und D, Ursolsäure, Hyptatsäure A, Zeorin, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, des weiteren Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien 3,20-dion, Bilobol, Ginkgol, Ginkgolsäure, Helenalin, Indicin, Indicin-N-oxid, Lasiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Oxoushinsunin, Aristolactam-All, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B und schwefelhaltige Aminosäuren wie Cystin sowie Salze, Hydrate, Solvate, Enantiomere, Racemate, Enantiomerengemische, Diastereomerengemische und Mischungen der vorgenannten Wirkstoffe.

Die erfindungsgemäßen Verfahren sind zur Beschichtung von beispielsweise Gefäßstützen und insbesondere Stents wie beispielsweise Koronarstents, Vaskularstents, Tracheastents (Luftröhrenstent), Bronchialstents, Harnröhrenstents, Speiseröhrenstents (Ösophagusstent), Gallenstents, Nierenstents, Dünndarmstents, Dickdarmstents geeignet. Zudem können Führungsdrähte, Spiralen, Katheter, Kanülen, Schläuche sowie allgemein röhrenförmige Implantate oder Teile der vorgenannten Medizinprodukte erfindungsgemäß beschichtet werden, wenn ein mit einem Stent vergleichbares Strukturelement in einem solchen Medizinprodukt enthalten ist. Sofern expandierbare Medizinprodukte bzw. Gefäßstützen eingesetzt werden, erfolgt die Beschichtung vorzugsweise im expandierten Zustand.

Die beschichteten Medizinprodukte werden insbesondere zur Offenhaltung aller gangartigen Strukturen eingesetzt, beispielsweise von Harnwegen, Speiseröhren, Luftröhren, Gallenwegen, Nierenwegen, Blutgefäßen im gesamten Körper einschließlich Gehirn, Duodenum, Pilorus, Dünn- und Dickdarm aber auch zum Offenhalten künstlicher Ausgänge wie sie für den Darm oder für die Luftröhre verwendet werden.

Somit sind die beschichteten Medizinprodukte zur Verhinderung, Verminderung oder Behandlung von Stenosen, Restenosen, Arteriosklerose, Atherosklerose, und allen anderen Formen des Gefäßverschlüsses oder Gefäßverengungen von Durchgängen oder Ausgängen geeignet.

Die vollflächige Beschichtung mit Polymer B geht über die Länge der Gefäßstütze hinaus und schließt nicht mit den Enden der Gefäßstütze ab.
Die überstehende Hülle wird in einem weiteren Schritt um die Ränder der Gefäßstütze zur Außenseite gelegt und die entstehenden Kanten unter Druck und erhöhter Temperatur in die darunterliegende Polymerschicht B integriert. Damit ist eine durchgängige Beschichtung auch der Ränder der Gefäßstütze gewährleistet und die Gefahr der Ablösung an diesen Schwachstellen ist nicht gegeben. Ausserdem lässt sich auf diese Weise unter den Rand eine Zugvorrichtung einbringen, mit der der Stent jederzeit sicher wieder entfernt werden kann. So lässt sich in der Faltung ein polymerer Faden umlegen, der an einer oder zwei gegenüberliegenden Seiten durch die Polymerschicht als Schlinge aus dem Rand nach außen ragt.
Eine weitere Möglichkeit bietet sich in der Nutzung dieses Randbereiches als Reservoir für Wirkstoffe bzw. zum Einbringen von Wirkstoffen speziell in diesen Randbereich, die sich durchaus von in/auf dem vollflächig beschichteten Hohlkörper eventuell vorhandenen Wirkstoffen unterscheiden.

Zusammenfassend kann gesagt werden, dass sich überraschenderweise gezeigt hat, dass es zur Beschichtung von Gefäßstützen und insbesondere von Stents aber auch von anderen biostabilen wie biodegradierbaren stentähnlichen Medizinprodukten, ein Verfahren gibt, mit dem die beschriebenen Nachteile des Standes der Technik verhindert werden. Das besonders problematische Ablösen der Hülle, das Fixieren im Gefäß, das Durchstoßen der Schicht an den Rändern und den die Gewebsirritationen verursachenden Kontakt zwischen Metall und Gefäßwand lassen sich mit diesem erfindungsgemäßen Verfahren vermeiden. Dabei erhält die den Stent umgebende Hülle die Flexibilität des Stents, trägt aber gleichzeitig zur mechanischen Festigkeit des Medizinproduktes bei. Es besteht mit diesem zusätzlich die Möglichkeit gezielt Wirkstoffe seitenspezifisch einzubringen, so z.B. ein Cytostatikum, dass von der äußeren Oberfläche in die Gefäßwand diffundieren kann und beispielsweise ein Antibiotikum in der Innenfläche des Medizinproduktes Infektionen verhindert. Zudem lassen sich zusätzlich durch die Möglichkeit der unterschiedlichen Beschichtung der Innen- und Außenseite weitere Optimierungen in der Anpassungen an die vor Ort herrschenden physiologischen Bedingungen realisieren.

Weitere Additive sind möglich, z.B. Substanzen, wie Bariumsulfat oder Edelmetalle, die ein implantiertes derart beschichtetes Medizinprodukt röntgenographisch sichtbar machen können. Des weiteren lassen sich die Außenfläche und die Innenfläche mit unterschiedlichem Material ummanteln wie oben ausgeführt. So lässt sich beispielsweise ein Medizinprodukt herstellen, dass auf der Außenseite eine hydrophobe Polymerhülle besitzt, während die Innenfläche aus hydrophilem Polymer besteht.

Dieses Verfahren bietet daher eine Vielfalt von Möglichkeiten, Medizinprodukte durch Variation und Kombination jedwede biostabile und biodegradierbare BeschichtungsMaterialien mit und ohne Additive auf Medizinprodukte im Bedarfsfall in Form einer Hülle aufzubringen.

Das Problem der möglichen Ablösung vom Medizinprodukt wird verhindert, indem man den Stent nicht wie in WO2005/030086 beschrieben vom Innenraum aus vollständig beschichtet, sondern die polymere Haut von der Außenseite anbringt. Obwohl die Beschichtung von außen aufgebracht wird, kann die Stentinnenseite im Bedarfsfall glatt und mit einer lückenlosen Polymerschicht ausgekleidet werden, während die beschichtete Außenseite ebenfalls einen lückenlos durchgängigen Überzug besitzt. Die Unebenheiten der Oberflächenstruktur, beim Stent in Form der Stentstreben, bleiben als Unebenheit unter der Beschichtung erhalten und heben sich hervor, ohne dass es in der Beschichtung an irgendeiner Stelle zu Lücken kommt. So wird trotz beidseitigem und vollflächigem Überzug die Fixierung im Gefäß ermöglicht.

Mit Hilfe des hier beschriebenen Verfahrens wird erreicht, dass zum einen das Ablösen der Beschichtung nicht möglich ist, da die Beschichtung mit dem Implantat eine feste Einheit bildet und zum anderen die Fixierung des beschichteten Stents auch unter den extremsten Bedingungen gewährleistet wird. Gewebsirritationen können nicht auftreten, da das Metall keinen Kontakt zum Gewebe hat.

Jedes Implantat unabhängig von Form und Material kann auf diese Weise vollständig oder unvollständig mit einem durchgängigen Überzug versehen werden.
Wichtig ist, dass mit Hilfe dieses Verfahrens der Überzug ein integraler Bestandteil des Implantates wird, so dass unter keinen Umständen eine Separierung von Polymerschicht und Stent bzw. eine Ablösung der Polymerschicht vom Stent erfolgen kann. Gleichzeitig kann der Überzug zur mechanischen Festigkeit eines Implantates beitragen ohne seine Flexibilität zu beeinträchtigen.

Das erfindungsgemäße Verfahren zur vollständigen flächendeckenden Beschichtung wird bevorzugt in drei voneinander getrennten Verfahrensschritten angewendet.
Im ersten Schritt werden nur die Bestandteile des Implantates und nicht die Freiräume gleichmäßig und in dünner Schicht mit einer Lösung, die das ausgewählte Polymere A enthält, beschichtet. Diese Beschichtung ist wichtig für den nächsten Schritt des vollflächigen Beschichtens.

Das Polymer kann synthetischer oder natürlicher Herkunft sein oder auch aus Mischungen aus verschiedenen biodegradierbaren und/oder biostabilen Polymeren bestehen. Die Beimischung eines oder mehrerer Wirkstoffe, die den Heilungsprozess durch deren zeitkontrollierte Freisetzung unterstützen können, lassen sich zufügen. Geeignete Wirkstoffe sind je nach Erkrankung auszuwählen, z.B. Entzündungshemmer, Cytostatika und Cytotoxika, Antibiotika, Antithrombotika, Antimykotika, Fungizide sowie die oben genannten Wirkstoffe.

Der zweite Schritt, indem das mit Polymer A beschichtete Implantat oder Implantatsegment flächendeckend überzogen wird, erfolgt als Tauchprozess, über Electrospinning oder Mikropipettierung.
Dazu wird das Implantat bzw. Medizinprodukt auf ein in Größe und Form passendes Formwerkzeug wie ein Fingerling auf einen Finger aufgezogen, doch so, dass es nicht verrutschen kann. Es darf auch nicht so fest sitzen, dass es deformiert wird. Die Wahl des selbstverständlich nicht rostenden Materials des Beschichtungswerkzeuges ist dabei nicht unwichtig, da die Materialeigenschaften des Werkzeuges beispielsweise das Verdunsten des Lösungsmittels aus der Lösung mit beeinflusst.
Bei Nutzung des Mikropipettierprozesses und Electrospinning besteht im Vergleich zum Tauchverfahren die Möglichkeit Zwischenräume nicht benachbarter Segmente des Medizinproduktes mit Polymer B zu bedecken. Das Electrospinning ermöglicht neben der bisher beschriebenen Erzeugung glatter Oberflächen auch die Strukturierung der Oberfläche innen oder/und außen in Form von fein- bis grobmaschigen Netzwerken in den Zwischenräumen des Implantates bzw. Segmentes.

Der Beschichtungsprozess mit Polymer B selbst ist vorzugsweise zweistufig. Zuerst wird das auf dem Werkzeug aufgebrachte Implantat oder das zu beschichtende Segment des Implantates mit dem reinen Lösungsmittel bzw. in eine stark verdünnte Lösung des Polymers, dass als Überzug vorgesehen ist, benetzt. Dazu wird das Tauchverfahren bevorzugt.
Die vorhandene Polymerbeschichtung A dient gleichfalls als Korrosionsbarriere zwischen metallischem Implantat und einem metallischem Coverwerkzeug.

Zum einen lassen sich aus dieser Lösung die beim Eintauchen eines Körpers in eine Flüssigkeit sich bildenden Luftblasen in der nicht viskosen Lösung entfernen, zum anderen wird so die Haftung der eigentlichen viskosen Beschichtungslösung durch Anlösen von Polymer A erhöht.

Essentiell für den zweiten Beschichtungsschritt ist der durch die Beschichtung mit Polymer A erreichte Abstand zwischen Implantat und Werkzeug, da nur so die Coverlösung enthaltend Polymer B durch Kapillarwirkung an die Innenseite des Implantats gelangt und sich auf der Oberfläche des Beschichtungswerkzeug in den freien Zwischenräumen der anliegenden mit Polymer A beschichteten Implantatelemente flächendeckend ausbreiten kann.

Das Implantat erhält damit die auf der Innenseite die erforderliche glatte einheitliche Fläche. An der Außenseite dagegen heben sich unter der Polymerschicht die Bestandteile des Implantats hervor, was und auch unter den extremen Bedingungen, die z.B. im Ösophagus oder in der Trachea entstehen können, dem Implantat Halt gibt.
Mit diesem Verfahren ist es zudem machbar, die Außenseite mit einem anderen polymeren Material zu versehen, als die Innenseite. So lässt sich z.B. für den Trachea-Stent die Innenseite zusätzlich mit einem hydrophilen Polymer überziehen. Damit kann man den Schleimabfluß noch verbessern.

Zusätzlich hat man auch hier die Möglichkeit, geeignete Wirkstoffe in den Überzug mit einzubringen, die ebenfalls auf der Außenseite andere sein können als auf der Innenseite.

Um noch zu verhindern, dass es an den Implantatenden zu Verletzungen kommen kann, ist für Medizinprodukte, deren vollflächige Beschichtung an den Implantatenden notwendig ist, ein weiterer Schritt vorteilhaft. Der erhaltene Überzug wird nicht in Höhe des Implantates gekürzt, sondern es wird an beiden Enden ein Überstand gelassen, der nun in diesem letzten Arbeitsgang um den Implantatrand umgelegt wird. Unter der Einwirkung von Druck und Wärme wird der umgeschlagene Überstand in die darunter liegende Schicht integriert. Anschließend ist keine Schnittkante mehr zu erkennen. Der umgelegte Rand und der darunter liegende Überzug sind zu einem nicht mehr voneinander zu unterscheidenden Verbund geworden. Nur so besteht keine Gefahr mehr, dass sich der Überzug am Rande lösen kann. Ein weiterer Vorteil ist, dass der dickere Überzug sich nicht so leicht beschädigen lässt. Dies kann immer wieder geschehen, wenn z.B. ein Trachea-Stent bei einem Husten so mitbewegt wird, dass Strebenenden durch die dünnere Beschichtung stoßen können. Als Folge kommt es zu Verletzungen an der Trachea durch den freigelegten Endpunkt aber auch zu einer Schwachstelle in der Beschichtung, die weitere Probleme nach sich ziehen kann. Zusätzlich zur erreichten erhöhten Sicherheit ermöglicht die dickere Randschicht auch das Anbringen von Vorrichtungen, wie sie zum Beispiel zur Entfernung des Implantats notwendig sein können und unter den Rand mitgelegt werden und damit mitversiegelt werden.
Unter den Rand des umgeschlagenen Überzuges lassen sich auch Wirkstoffe einbringen, die dann aus diesem Depot langsam entweichen.

Ebenso besteht die Möglichkeit zur Verbesserung der Röntgensichtbarkeit beispielsweise dadurch, Schwermetallsalze oder Edelmetalle in den dickeren Rändern aber auch im gesamten Überzug einzubringen, ohne dass sich Lokalelemente bilden können, die bei direktem Kontakt zwischen den Metallen ohne weiteres entstehen können.

Somit hat sich gezeigt, dass die hierin beschriebenen Verfahren die oben beschriebenen Nachteile von bekannten Endoluminalprothesen vollständig beheben. Dabei sind die Variationsmöglichkeiten bezüglich Form und Material des Implantats sowie des biokompatiblen Beschichtungsmaterial und Wirkstoffe unbegrenzt und ergeben sich vor allem aus sinnvollen dem Patienten zugute kommenden Kombinationen.

So ist beispielsweise bis heute der Einsatz von Stents zum Zurückdrängen von Gallengangkarzinomen nicht üblich. Doch kann nur in ca. 10% der Fälle die chirurgische Entfernung durchgeführt werden. Die Lebenserwartung dieser Patienten liegt bei durchschnittlich 1 Jahr. Der Einsatz eines mit Hilfe dieses Verfahrens vollständig beschichteten und für den Gallengang geeigneten Implantates, welches z.B. ein Chemotherapeutikum enthält, könnte zum einen die Verengung des Körperdurchganges durch Gegendruck der Gefäßstütze verhindern und gleichzeitig die Tumorbildung verlangsamen oder sogar stoppen und würde somit zumindest eine lebensverlängernde Maßnahme bei hoher oder guter Lebensqualität darstellen.

Ferner lässt sich die erfindungsgemäße Beschichtung auch im vaskulären System nutzen. Bei der Aneurysmenbildung beispielsweise so, dass die Beschichtung verhindert, dass sich das Aneurysma durch weitere Blutzufuhr vergrößern kann

Als weiteres Beispiel kann ein solches Medizinprodukt als Grundlage für Tissue engineering dienen, wofür sich vor allem das Electrospinning zur Beschichtung anbietet.

### Beispiele

### Beispiel 1: Vorbeschichtung von Stents mit Sprühvorgang

An den Stab eines Rotators wird ein Stent befestigt und bei geringer Drehzahl mit sehr langsamen Auf- und Abbewegungen der Pistole wird der Stent mit einer 1%igen Polyurethanlösung eingesprüht.. Durch das Aufsprühen wird der Stent gräulich-matt, so dass man eine optische Sprühkontrolle durchführen kann. Besonders wichtig ist es, dass der Rand einwandfrei besprüht ist, was durch ein zusätzliches Sprühen rundherum gewährleistet wird. Danach lässt man trocknen.

### Beispiel 2: Polysulfon-Sprühlösung:

176 mg PS (Polyethersulfon, Odel®, erhältlich von Solvay) werden eingewogen und mit Chloroform auf 20 g aufgefüllt. (0,88 % PS -Lösung) und wie in Beispiel 1 auf den Stent gesprüht.

### Beispiel 3: Vollflächige Beschichtung eines gesprühten Stents durch Tauchbeschichtung

Polyurethan wird in THF gelöst, so dass man eine 14 %ige Lösung erhält. Ein nach Beispiel 1 vorbeschichteter Stent wird vorsichtig auf das passende Formwerkzeug aufgeschoben.

Das Werkzeug mit dem aufgezogenen Stent wird kopfüber in reines THF getaucht bis Luftblasen erscheinen, die aufsteigen. Danach taucht man langsam in die 14%ige Polyurethan-Lösung. Nach 15 Sekunden zieht man den Kern wieder langsam heraus und bringt ihn sofort in die Waagerechte und dreht den Kern, damit sich das PU gleichmäßig auf dem Stent verteilt und trocknet.

Wenn es nicht mehr verläuft, wird der Kern unter dem Abzug trocknen gelassen und anschliessend für 45 min bei 95°C im Trockenschrank getempert. Nach dem Abkühlen wird in eine warme 0,3%ige SDS-Lösung eingetaucht, um den Stent vom Werkzeug abzulösen. Nach Reinigung unter fließendem Wasser und Spülen mit 0,5 m NaOH wird sehr gründlich unter fließendem lauwarmem Wasser und in VE-Wasser gespült.

### Beispiel 4: Vollflächige Beschichtung eines gesprühten Stents mit PU/Tergurid durch Tauchbeschichtung

Die Tauchlösung besteht aus einer 30Gew-% Tergurid in Polymer, die dann mit THF auf 10% verdünnt wird. Die weitere Handhabung erfolgt wie in Beispiel 2.

### Beispiel 5: Vollflächige Beschichtung eines gesprühten Stents mit PU/ Cyclosporin durch Tauchbeschichtung

Die Tauchlösung besteht aus einer 30Gew-% Cycloporin A in Polymer, die dann mit THF auf 10% verdünnt wird. Die weitere Handhabung erfolgt wie in Beispiel 2.

### Beispiel 6: Beschichtung eines vollflächig beschichteten Stents mit Paclitaxel nach dem Sprühverfahren

Die nach Beispiel 1 und Beispiel 2 mit einem Polymer vollflächig beschichteten Stents werden horizontal auf eine dünne Metallstange gehängt, die auf die Rotationsachse der Rotations- und Vorschubanlage gesteckt ist und mit 10 U/min rotiert. Die Stents werden so aufgebracht, das die Innenseite des Stents die Stange nicht berührt. Bei einer Vorschubamplitude von 2,2,cm und einer Vorschubgeschwindigkeit von 4cm/s und einem Abstand von 8 cm zwischen Stent und Düse wird der Stent mit der jeweiligen Sprühlösung besprüht. Nach dem Trocknen (ca. 15 min) bei Raumtemperatur und folgend im Abzug über Nacht wird erneut gewogen.

Herstellung der Sprühlösung : 44 mg Taxol werden in 6g Chloroform gelöst.

### Beispiel 7: Bördeln (Fold-over) des Randes des PU-gecoverten Stents

Der überstehende Rand der Polymerhülle wird mit Ethanol abgespritzt, damit das PU nicht aufeinander klebt. Danach wird der PU-Rand über den rundherum um den Stentrand zurückgeschlagen. Der Rand wird solange geglättet., bis keine Falten mehr zu sehen sind. Bei dem ganzen Vorgang ist darauf zu achten, dass der Rand nicht zu stramm umgeschlagen wird (keine Krönchenstruktur!), da sich sonst die Stentenden durch das Covering drücken können und Löcher entstehen.

### Beispiel 8: Nutzung des Randes als Wirkstoffreservoir

Der überstehende Rand der Polymerhülle an beiden Enden des vollflächig beschichteten Stents wird wie in Beispiel 6 beschrieben zurückgeschlagen und vor dem Glätten mit 200µl einer Lösung aus 30Gew.% Fasudil in Ethanol/Wasser (50:50 v:v) befüllt und anschliessend so angetrocknet, dass in wie in Beispiel 6 weiterverfahren werden kann.

### Beispiel 9: Anbringen einer Zugvorrichtung zur Entfernung des Stents

Hierzu wird ein zugfester Polyurethanfaden rund um den überstehenden Polymerrand des nach Beispiel 3 beschichteten Hohlkörpers gelegt, so dass sich Anfang und Ende des Fadens gerade treffen und der Faden nach dem Bördeln wie in Beispiel 7 beschrieben, knapp unter dem Stentende zu liegen kommt. Für die Schlaufenbildung wird im 90° Winkel vom Fadenanfang mit einer Nadel durch die überstehende Polymerhülle der Faden in den Innenbereich geführt und in genügendem Abstand vom ersten Durchstich wieder auf die Außenseite gebracht, so dass ein Henkel entsteht, der im 90°Winkel zur Stentlängsachse steht und zum Kreismittelpunkt zeigt. Desgleichen wird bei einem Winkel von 270° vom Fadenanfang verfahren, bevor das Fadenende den Fadenanfang erreicht. Das darauf folgende Umlegen des Randes bringt die beiden Schlaufen aus der zur Längsachse im rechten Winkel stehenden Position in Parallellage zur Längsachse, so dass die Polymerschlaufen an diesen zwei Stellen eine schlaüfenförmige Verlängerung des beschichteten Hohlkörpers darstellen. Mit dem im Beispiel 10 beschriebenen Sealen des umgelegten Randes wird der PU-Faden in den Rand fest integriert.

### Beispiel 10 : Sealen des Fold-Overs

Der umgefaltete Rand wird mit Druck und Hitze versiegelt. Dazu wird der Stent wieder auf das Formwerkzeug gezogen. Je eine mit Silikonschaumstreifen ausgelegte Schlauchschelle wird über die Stentenden geschoben und mit dem Drehmomentschlüssel angezogen. Dann kommen die Stents für mindestens 4 Stunden bei 95°C in den Trockenschrank. Nach dem Abkühlen werden die Klemmen gelöst und der Stent wie in Beispiel 3 mittels Benetzen im SDS-Bad abgelöst, gereinigt und luftgetrocknet.

### Beispiel 11: Hydrophile Beschichtung der luminalen Seite eines vollflächig beschichteten Stents

Die Lösungsmittel Isopropanol, Methylethylketon und Diacetonalkohol werden im Volumen-Verhältnis 1:2:1 gemischt und mit PVP zu einer 35%igen Lösung verrührt. Der vollflächig polymerbeschichtete Stent wird auf einen Metallkonus aufgesetzt, so dass die PVP-Lösung zwischen Konus und Stent eingefüllt werden kann. Dabei ist darauf zu achten, dass nichts über den oberen Rand herunterläuft, da nur die Innenseite beschichtet werden soll. Nach einer kurzen Wartezeit wird der Stent angehoben und die Lösung läuft über den Konus ab. Man lässt den Stent gut abtropfen.

Trocknen und Vernetzen: Nach dem Trocknen im Trockenschrank werden die Stents in die UV-Kammer senkrecht eingestellt, so dass das Licht von oben in den Stent hineinleuchten kann und bei 500 Watt bestrahlt. Anschließend wird überschüssiges nicht vernetztes PVP durch sorgfältiges Spülen der Stents mit Wasser entfernt.

### Beispiel 12: Partielle Beschichtung von Stents (d=3mm)

Lösung: 3,2 mg PU gelöst in 20ml N-Methyl-2-Pyrrolidon

Ein sprühbeschichtete Stent wird auf ein passendes frei drehbares Formwerkzeug geschoben, so das er vollständig auf dem glatten Untergrund aufliegt.

Das Auftragen der Beschichtung erfolgt in mindestens zwei Schichten, wobei mit einem Pinselhaar Lösung aufgenommen und diese in das zu beschichtende Feld aufgetragen wird, bis das Feld komplett mit Lösung bedeckt ist.

Wenn jedes ausgewählte zu beschichtende Feld in der gewünschten Beschichtungsdicke gefüllt ist, wird der Stent bei 90°C getrocknet. Nach dem Abkühlen wird der Stent vom Formwerkzeug gelöst.

### Beispiel 13: Beschichtung eines Stentsegmentes mit einem engmaschigen Netzwerk aus Polyethersulfonfäden mittels Electrospinning

Lösung : 20Gew.-% Polyethersulfon in Methylenchlorid

Die Lösung wird in die Glaspipette der Electrospinning-apparatur gefüllt. Es wird eine Spannung von 12 kV angelegt und bei einer Flussrate von 3ml/h wird der sich bildende Polyethersulfonfaden auf das zu beschichtende Stent-Segment gerichtet, der sich in einem Abstand von 25 cm befindet und beginnend von einer Stentstrebe zur nächsten beschichtet.

### Beispiel 14: Vollflächige Beschichtung eines Stents mittels Grenzflächenpolykondensation

Das für die Grenzflächenpolykondensation verwendete Formwerkzeug besteht aus einem Stab oder Stift der an einem Ende konisch zuläuft, während das andere Ende eben ist, so dass man das Werkzeug standfest und aufrecht stellen kann. Am spitz zulaufenden Ende, dass während der Beschichtung nach oben zeigt, ist ein dünner Draht angebracht, der mit einer Hebeapparatur verbunden ist, so dass der Stent senkrecht nach oben gezogen werden kann.

Der Stent wird vorsichtig auf das passende Formwerkzeug gebracht und senkrecht in die Mitte des Reaktionsbehälters gestellt, dass eine Lösung von 15 mL Adipinsäuredichlorid in 500 ml Chloroform enthält. Das Formwerkzeug muss vollständig in der unteren Lösung stehen. Lediglich der dünne Draht, der an der Hebeapparatur befestigt wird, ragt aus dem Behälter. Danach wird die Lösung langsam mit einer Mischung aus 22 g α,ω-1,6-Hexamethylendiamin und 40 g Natriumcarbonat in 600 mL Wasser überschichtet. An der Phasengrenze bildet sich der Polymerfilm. Nun wird der Stent langsam und gleichmässig herausgezogen, so dass sich der dabei entstehende Schlauch um den Stent legen kann. Die Kondensationsreaktion wird ca. 1 cm nach Beschichten des Stentendes durch Unterbrechung der Aufwärtsbewegung gestoppt. Der derart umhüllte Stent wird anschließend mit 50%igem Ethanol gespült, anschließend mit Wasser gründlich gewaschen und im Trockenschrank bei 30°C getrocknet.

### Beispiel 15: Kovalente Beschichtung eines Stents mit einem Heparinderivat zur hämokompatiblen Ausrüstung der Stentoberfläche

Nicht expandierte Stents aus medizinischem Edelstahl LVM 316 wurden im Ultraschallbad mit Aceton und Ethanol entfettet und bei 100°C im Trockenschrank getrocknet. Danach wurden sie für 5 Minuten in eine 2%ige Lösung von 3-Aminopropyltriethoxysilan in einem Gemisch Ethanol/Wasser (50/50 : (v/v)) getaucht und anschließend bei 100°C getrocknet. Anschließend wurden die Stents mit demineralisiertem Wasser gewaschen.

3 mg desultatiertes und reacetyliertes Heparin wurden bei 4°C in 30 ml 0,1M MES-Puffer (2-(N-Morpholino)ethansulfonsäure) pH 4,75 gelöst und mit 30 mg N-Cyclohexyl-N'-(2-morpholinoethyl)carbodümid-methyl-p-toluolsulfonat versetzt. In dieser Lösung wurden die Stents für 15 Stunden bei 4°C gerührt. Anschließend wurde mit Wasser, 4M NaCl-Lösung und Wasser für je 2 Stunden gespült.

### Beispiel 16: Tauchbeschichtung eines Stents mit einer Silikonhülle zum Einsatz als künstlicher Darmausgang

Ein Stent, der auf einem passenden Formwerkzeug aufgebracht wird, wird gemäß Beispiel 2 in eine käufliche in N-Methylpyrrolidon gelösten Silicon-Block-PolymerLösung (Gelest Sibrid®) beschichtet. Anschließend wird das Lösungsmittel bei 75°C im Trockenschrank vollständig entfernt und der beschichtete Stent vorsichtig mit warmem Wasser vom Werkzeug abgelöst. Der überstehende Rand wird wie in Beispiel 2 und 3 beschrieben unter Benetzung mit Ethanol oder THF umgelegt und mit dem Stent verbunden.

### Beispiel 17: Beschichtung eines Stents mit einer Silikonhülle

Der nach Beispiel 1 beschichtete Stent wird auf ein passendes Formwerkzeug gebracht und mit einer Präpolymersilikondispersion die nach dem Acetoxy-Vernetzersystem polykondensiert (z.B. MED1-6604; MED-6605 oder MED6-6606 von NuSil) sprühbeschichtet. Zuvor wird die Dispersion mit einem geeigneten Lösungsmittel (z.B. mit n-Hexan, THF, Chloroform, Dichlormethan, Aceton, Ethanol, Ether) auf eine zur Sprühbeschichtung geeigneten Verdünnung gebracht (ca. 1-2Gew% Polydimethylsiloxanpräpolymer im LM). Nach dem Sprühprozess wird der Stent bei Raumtemperatur und einer rel. Luftfeuchtigkeit von mindestens 30% getrocknet, während dessen die Polykondensation der Präpolymere je nach vorhandener funktioneller Gruppe unter Abspaltung der entsprechenden niedermolekularen Carbonsäure unter Bildung der Silikonhülle stattfindet.

Nach Abschluss des Trocknungsprozesses wird der mit Silikon beschichtete Stent mit wenig Wasser vom Formwerkzeug gelöst und anschliessend mit so viel Wasser solange gewaschen, bis der pH-Wert von Wasser erreicht wird.

### Beispiel 18: Ermittlung des Elutionsverhaltens von mit Wirkstoff beladenen Stent in PBS-Puffer

Je nach Größe des Stent wird dieser in einem genügend kleinen Gefäß mit PBS-Puffer versetzt, so dass er vollständig in PBS liegt. Danach wird mit Parafilm verschlossen und im Trockenschrank bei 37°C inkubiert. Nach Ablauf der gewählten Zeitintervalle wird jeweils der Überstand abpipettiert und dessen UV-Absorption bei 306 nm gemessen.

### Beispiel 19: Vollflächige Tauchbeschichtung eines Stents mit einem bioabbaubaren Polymeren

Der nach Beispiel 1 sprühbeschichtete Stent wird auf ein Formwerkzeug gebracht und wie in Beispiel 3 beschrieben in eine 15Gew.%ige Polylactid-Chloroform-Tauchlösung gebracht und beschichtet. Anschliessend wird der Stent bei Raumtemperatur an der Luft getrocknet und durch Benetzen mit Aceton vom Formwerkzeug gelöst.

### Beispiel 20: Vollflächige Sprühbeschichtung eines Stens mit einem bioabbaubaren Polymeren und einem Wirkstoff

Der nach Beispiel 1 mit sprühbeschichtete Stent wird auf ein Formwerkzeug gebracht und gleichmäßig von allen Seiten mit der Rapamycin-Polylaktid-Glycolid-Lösung eingesprüht und bei Raumtemperatur an der Luft getrocknet. Dieser Vorgang wird mindestens zwei mal wiederholt. Anschliessend wird der vollflächig beschichtete Stent vom Formwerkzeug gelöst.
Sprühlösung :
22 mg PLGA und 22 mg Rapamycin werden eingewogen und mit Chloroform auf 5 g aufgefüllt.

## Patentansprüche

1. Verfahren zur vollflächigen Beschichtung von gitterartigen oder netzartigen Gefäßstützen umfassend die folgenden Schritte:
a) Bereitstellung einer nicht vollflächigen, gitterartigen oder netzartigen Gefäßstütze mit Zwischenräumen zwischen den das Gitter oder Netz ausbildenden Streben,
b) zumindest teilweise Beschichtung der Streben mit einem Polymer A,
c) Benetzung der Oberfläche der mit dem Polymer A beschichteten Gefäßstütze mit einem organischen Lösungsmittel,
d) vollflächige Beschichtung der Zwischenräume zwischen den die gitterartige oder netzartige Struktur ausbildenden Streben mit einer polymeren Beschichtung eines Polymers B, wobei die vollflächige Beschichtung mit Polymer B über die Länge der Gefäßstütze hinausgeht und in einem weiteren Schritt um die Ränder der Gefäßstütze zur Außenseite gelegt und die entstehenden Kanten unter Druck und erhöhter Temperatur in die darunterliegende Schicht des Polymers B integriert wird.

2. Verfahren zur vollflächigen Beschichtung von gitterartigen oder netzartigen Gefäßstützen gemäß Anspruch 1 umfassend die folgenden Schritte:
a) Bereitstellung einer nicht vollflächigen, gitterartigen oder netzartigen Gefäßstütze umfassend Streben mit einer inneren Oberfläche und einer äußeren Oberfläche mit Zwischenräumen zwischen den einzelnen Streben,
b) zumindest teilweise Beschichtung der inneren als auch der äußeren Oberfläche der Streben mit einem Polymer A,
c) Benetzung der Oberfläche der mit dem Polymer A beschichteten Gefäßstütze mit einem organischen Lösungsmittel,
d) vollflächige Beschichtung der inneren und/oder äußeren Oberfläche sowie der Zwischenräume zwischen den Streben mit einer polymeren Beschichtung eines Polymers B, wobei die vollflächige Beschichtung mit Polymer B über die Länge der Gefäßstütze hinausgeht und in einem weiteren Schritt um die Ränder der Gefäßstütze zur Außenseite gelegt und die entstehenden Kanten unter Druck und erhöhter Temperatur in die darunterliegende Schicht des Polymers B integriert wird.

3. Verfahren nach Anspruch 1 oder 2, wobei Polymer A und Polymer B identisch sind.

4. Verfahren nach Anspruch 1 oder 2, wobei der Beschichtungsschritt b) mittels Sprühverfahren oder Electrospinning ausgeführt wird.

5. Verfahren nach Anspruch 1 oder 2, wobei bei dem Beschichtungsschritt b) die Zwischenräume zwischen den Streben nicht mit einer polymeren Schicht überzogen werden.

6. Verfahren nach Anspruch 1 oder 2, wobei die Benetzung gemäß Schritt c) im Tauchverfahren oder Sprühverfahren durchgeführt wird.

7. Verfahren nach einem der Ansprüche 1 - 6, wobei das zur Benetzung verwendete organische Lösungsmittel gemäß Schritt c) das Polymer B in einer Konzentration enthält, welche unter der Konzentration des Polymers B in der Lösung gemäß Schritt d) liegt.

8. Verfahren nach einem der Ansprüche 1 - 7, wobei das Polymer A und/oder das Polymer B aus der Gruppe ausgewählt wird umfassend Polyacrylsäure und Polyacrylate wie Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyvinylaromaten, Polyvinylester, Polyvinylpyrollidone, Polyoxymethylene, Polyethylen, Polypropylen, Polytetrafluorethylen, Polyurethane, Polyolefin-Elastomere, Polyisobutylene, EPDM-Gummis, Fluorosilicone, Carboxymethylchitosan, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetat-butyrate, Ethylvinylacetat-copolymere, Polysulfone, Polyethersulfone, Epoxyharze, ABS-Harze, EPDM-Gummis, Silicone wie Polysiloxane, Polyvinylhalogene und Copolymere, Celluloseether, Cellulosetriacetate, Chitosan, Chitosanderivate, polymerisierbare Öle wie z.B. Leinöl und Copolymere und/oder Mischungen derselben.

9. Verfahren nach einem der Ansprüche 1 - 7, wobei das Polymer A und/oder das Polymer B aus der Gruppe ausgewählt wird umfassend Polyvalerolactone, Poly-ε-Decalactone, Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäureanhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere wie z.B. aus Oligocaprolactondiole und Oligodioxanondiole, Polyetherester-multiblockpolymere wie z.B. PEG und Poly(butylenterephtalat. Polypivotolactone, Polyglycolsäuretrimethyl-carbonate Polycaprolacton-glycolide, Poly(g-ethylglutamat), Poly(DTH-Iminocarbonat), Poly(DTE-co-DT-carbonat), Poly(Bisphenol A-iminocarbonat), Polyorthoester, Polyglycol-säuretrimethylcarbonate, Polytrimethylcarbonate, Polyiminocarbonate, Poly(N-vinyl)-Pyrolidon, Polyvinylalkohole, Polyesteramide, glycolierte Polyester, Polyphosphoester, Polyphosphazene, Poly[p-carboxyphenoxy)propan] Polyhydroxypentansäure, Polyanhydride, Polyethylenoxid-propylenoxid, weiche Polyurethane, Polyurethane mit Aminosäurereste im Backbone, Polyetherester wie das Polyethylenoxid, Polyalkenoxalate, Polyorthoester sowie deren Copolymere, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, desweiteren Hyaluronsäure, Heparansulfat, Heparin, Chondroitinsulfat, Dextran, b-Cyclodextrine, und Copolymere mit PEG und Polypropylenglycol, Gummi arabicum, Guar, Gelatine, Collagen Collagen-N-Hydroxysuccinimid, Modifikationen und Copolymere und/oder Mischungen der vorgenannten Substanzen.

10. Verfahren nach einem der Ansprüche 1 - 9, wobei die Gefäßstütze mittels Tauchverfahren, Elektrospinning oder/und Mikropipettierung mit einer vollflächigen polymeren Beschichtung überzogen werden.

11. Verfahren nach einem der Ansprüche 1 - 10, wobei unter, in und/oder auf der Schicht aus dem Polymer A und/oder unter, in und/oder auf der Schicht aus dem Polymer B oder Teilbereichen dieser Schichten mindestens ein antiproliferativer, antimigrativer, antiangiogener, antiinflammatorischer, antiphlogistischer, zytostatischer, zytotoxischer und/oder antithrombotischer Wirkstoff aufgebraucht und/oder eingebracht wird.

12. Verfahren nach Anspruch 11, wobei der antiproliferativer, antimigrativer, antiangiogener, antiinflammatorischer, antiphlogistischer, zytostatischer, zytotoxischer und/oder antithrombotischer Wirkstoff ausgewählt wird aus der Gruppe umfassend: Sirolimus (Rapamycin), Everolimus, Pimecrolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid, Estramustin, Melphalan, Ifosfamid, Tropfosfamid, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, 8-α-Ergoline, Dimethylergoline, Agroclavin, 1-Allylisurid, 1-Allyltergurid, Bromergurid, Bromocriptin, Elymoclavin, Ergocristin, Ergocristinin, Ergocornin, Ergocorninin, Ergocryptin, Ergocryptinin, Ergometrin, Ergonovin, Ergosin, Ergosinin, Ergotmetrinin, Ergotamin, Ergotaminin, Ergovalin, Lergotril, Lisurid, Lysergol, Lysergsäure, Lysergsäureamid, Lysergsäurediethylamid, Isolysergsäure, Isolysergsäureamid, Isolysergsäurediethylamid, Mesulergin, Metergolin, Methergin, Methylergometrin, Methysergid, Pergolid, Protergurid und Tergurid, Celecoxip, Thalidomid, Fasudil^{®}, Ciclosporin, SMC-Proliferation-Inhibitor-2w, Epothilone A und B, Mitoxanthrone, Azathioprin, Mycophenolatmofetil, c-myc-Antisense, b-myc-Antisense, Betulinsäure, Camptothecin, PI-88 (sulfatiertes Oligosaccharid), Melanocyte-stimulating hormon (α-MSH), aktiviertes Protein C, IL1-ß-Inhibitor, Thymosin α-1, Fumarsäure und deren Ester, Calcipotriol, Tacalcitol, Lapachol, ß-Lapachon, Podophyllotoxin, Betulin, Podophyllsäure-2-ethylhydrazid, Molgramostim (rhuGM-CSF), Peginterferon α-2b, Lanograstim (r-HuG-CSF), Filgrastim, Macrogol, Dacarbazin, Basiliximab, Daclizumab, Selectin (Cytokinantagonist), CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, 1,11-Dimethoxycanthin-6-on, 1-Hydroxy-11-Methoxycanthin-6-on, Scopolectin, Colchicin, NO-Donoren wie Pentaerythrityltetranitrat und Syndnoeimine, S-Nitrosoderivate, Tamoxifen, Staurosporin, ß-Estradiol, α-Estradiol, Estriol, Estron, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A, Paclitaxel und dessen Derivate wie 6-α-Hydroxy-Paclitaxel, Baccatin, Taxotere, synthetisch hergestellte als auch aus nativen Quellen gewonnene macrocyclische Oligomere des Kohlensuboxids (MCS) und seine Derivate, Mofebutazon, Acemetacin, Diclofenac, Lonazolac, Dapson, o-Carbamoylphenoxyessigsäure, Lidocain, Ketoprofen, Mefenaminsäure, Piroxicam, Meloxicam, Chloroquinphosphat, Penicillamin, Tumstatin, Avastin, D-24851, SC-58125, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin, Tobramycin, Gentamycin, Penicilline wie Dicloxacillin, Oxacillin, Sulfonamide, Metronidazol, Antithrombotika wie Argatroban, Aspirin, Abciximab, synthetisches Antithrombin, Bivalirudin, Coumadin, Enoxoparin, desulfatiertens und N-reacetyliertes Heparin, Gewebe-Plasminogen-Aktivator, GpIIb/IIIa-Plättchenmembranrezeptor, Faktor Xₐ-Inhibitor Antikörper, Heparin, Hirudin, r-Hirudin, PPACK, Protamin, Natriumsalz der 2-Methylthiazolidin-2,4-dicarbonsäure Prourokinase, Streptokinase, Warfarin, Urokinase, Vasodilatoren wie Dipyramidol, Trapidil®, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon α, ß und γ, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol, Vitamin B1, B2, B6 und B12, Folsäure, Tranilast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, D24851, SC-58125, Retinolsäure, Quinidin, Disopyrimid, Flecainid, Propafenon, Sotolol, Amidoron, natürliche und synthetisch hergestellte Steroide wie Bryophyllin A, Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Antimykotika wie Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceanole A, B und C, Bruceantinoside C, Yadanzioside N und P, Isodeoxyelephantopin, Tomenphantopin A und B, Coronarin A, B, C und D, Ursolsäure, Hyptatsäure A, Zeorin, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, des weiteren Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien 3,20-dion, Bilobol, Ginkgol, Ginkgolsäure, Helenalin, Indicin, Indicin-N-oxid, Lasiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Oxoushinsunin, Aristolactam-All, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B und schwefelhaltige Aminosäuren wie Cystin sowie Salze, Hydrate, Solvate, Enantiomere, Racemate, Enantiomerengemische, Diastereomerengemische und Mischungen der vorgenannten Wirkstoffe.

13. Verfahren nach einem der Ansprüche 1 - 12, wobei die zu beschichtende Gefäßstütze eine röhrenförmige, spiralförmige und/oder geflochtene Struktur aufweist.

14. Verfahren nach einem der Ansprüche 1 - 12, wobei es sich bei der zu beschichtenden Gefäßstütze um einen Stent, Koronarstent, Vaskularstent, Trachealstent (Luftröhrenstent), Bronchialstent, Harnröhrenstent, Speiseröhrenstent (Ösophagusstent), Gallenstent, Nierenstent, Dünndarmstent, Dickdarmstent, Kehlkopfimplantat, Bypass, Katheter oder künstlichen Darmausgang handelt.

15. Gefäßstütze erhältlich nach einem Verfahren gemäß eines der Ansprüche 1 - 14.

16. Gefäßstütze gemäß Anspruch 15 geeignet zur Verhinderung, Verminderung oder Behandlung von Stenose, Restenose, Arteriosklerose, Atherosklerose, Gefäßverschlüssen, Gefäßverengungen, Aneurysmen, künstliche Ausgänge und Zugänge.

## Claims

1. Method for coating the entire surface of lattice-like or mesh-like endoprostheses, comprising the following steps:
a) providing a lattice-like or mesh-like endoprosthesis having a discontinuous surface with interstices between the struts forming the lattice or mesh,
b) at least partially coating the struts with a polymer A,
c) wetting the surface of the endoprosthesis coated with the polymer A with an organic solvent,
d) coating the entire surface of the interstices between the struts which form the lattice-like or mesh-like structure with a polymer coating of a polymer B, wherein the coating of a polymer B which covers the entire surface exceeds the length of the endoprosthesis and in a further step is placed around the edges of the endoprosthesis on the outer surface and the formed edges are being integrated into the subjacent polymer layer B under pressure and increased temperature.

2. Method for coating the entire surface of lattice-like or mesh-like endoprostheses according to claim 1, comprising the following steps:
a) providing a lattice-like or mesh-like endoprosthesis having a discontinuous surface and comprising struts with an inner surface and an outer surface with interstices between the individual struts,
b) at least partially coating the inner as well as the outer surface of the struts with a polymer A,
c) wetting the surface of the endoprosthesis coated with the polymer A with an organic solvent,
d) coating the entire surface of the inner and/or outer surface as well as of the interstices between the struts with a polymer coating of a polymer B, wherein the coating of a polymer B which covers the entire surface exceeds the length of the endoprosthesis and in a further step is placed around the edges of the endoprosthesis on the outer surface and the formed edges are being integrated into the subjacent polymer layer B under pressure and increased temperature.

3. Method according to claim 1 or 2, wherein polymer A and polymer B are identical.

4. Method according to claim 1 or 2, wherein the coating step b) is performed by spray coating or electrospinning.

5. Method according to claim 1 or 2, wherein the interstices between the struts are not being covered with a polymer layer in the coating step b).

6. Method according to claim 1 or 2, wherein the wetting according to step c) is performed by dip coating or spray coating.

7. Method according to any one of claims 1 - 6, wherein the organic solvent used for the wetting according to step c) contains the polymer B in a concentration lower than that of the polymer B in a solution used for coating according to step d).

8. Method according to any one of claims 1 - 7, wherein the polymer A and/or the polymer B is selected from the group comprising: polyacrylic acid and polyacrylates such as polymethylmethacrylate, polybutylmethacrylate, polyacrylamide, polyacrylonitriles, polyamides, polyetheramides, polyethylene amine, polyimides, polycarbonates, polycarbourethanes, polyvinyl ketones, polyvinyl halides, polyvinylidene halides, polyvinyl ethers, polyvinylarenes, polyvinyl esters, polyvinyl pyrrollidones, polyoxymethylenes, polyethylene, polypropylene, polytetrafluoro-ethylene, polyurethanes, polyolefine elastomers, polyisobutylenes, EPDM gums, fluorosilicones, carboxymethyl chitosans, polyethylene terephthalate, polyvalerates, carboxymethylcellulose, cellulose, rayon, rayon triacetates, cellulose nitrates, cellulose acetates, hydroxyethyl cellulose, cellulose butyrates, cellulose acetate butyrates, ethyl vinyl acetate copolymers, polysulphones, polyethersulphones, epoxy resins, ABS resins, silicones such as polysiloxanes, polyvinyl halogens and copolymers, cellulose ethers, cellulose triacetates, chitosan, chitosan derivatives, polymerizable oils such as linseed oil and copolymers and/ or mixtures thereof.

9. Method according to any one of claims 1 - 7, wherein the polymer A and/or the polymer B is selected from the group comprising: polyvalerolactones, poly-ε-decalactones, polylactides, polyglycolides, copolymers of the polylactides and polyglycolides, poly-ε-caprolactone, polyhydroxybutyric acid, polyhydroxybutyrates, polyhydroxyvalerates, polyhydroxybutyrate-co-valerates, poly(1,4-dioxane-2,3-diones), poly(1,3-dioxane-2-one), poly-para-dioxanones, polyanhydrides such as polymaleic acid anhydrides, polyhydroxymethacrylates, fibrin, polycyanoacrylates, polycaprolactone-dimethylacrylates, poly-β-maleic acid, polycaprolactonebutyl-acrylates, multiblock polymers such as from oligocaprolactonedioles and oligodioxanonedioles, polyetherester multiblock polymers such as PEG and poly(butyleneterephthalate), polypivotolactones, polyglycolic acid trimethyl-carbonates, polycaprolactone-glycolides, poly(γ-ethylglutamate), poly(DTH-iminocarbonate), poly(DTE-co-DT-carbonate), poly(bisphenol-A-iminocarbonate), polyorthoesters, polyglycolic acid trimethyl-carbonates, polytrimethylcarbonates, polyiminocarbonates, poly(N-vinyl)-pyrrolidone, polyvinylalcohols, polyesteramides, glycolated polyesters, polyphosphoesters, polyphosphazenes, poly[p-carboxyphenoxy)propane], polyhydroxypentanoic acid, polyanhydrides, polyethylene oxide-propylene oxide, soft polyurethanes, polyurethanes with amino acid residues in the backbone, polyetheresters such as polyethylene oxide, polyalkeneoxalates, polyorthoesters as well as copolymers thereof, carrageenanes, fibrinogen, starch, collagen, protein based polymers, polyamino acids, synthetic polyamino acids, zein, modified zein, polyhydroxyalkanoates, pectic acid, actinic acid, modified and unmodified fibrin and casein, carboxymethyl sulphate, albumin, furthermore hyaluronic acid, heparan sulphate, heparin, chondroitine sulphate, dextran, β-cyclodextrins, and copolymers with PEG and polypropylene glycol, gummi arabicum, guar, gelatine, collagen, collagen-N-Hydroxysuccinimide, modifications and copolymers and/ or mixtures of the substances mentioned above.

10. Method according to any one of claims 1 - 9, wherein the endoprosthesis is completely covered with a polymer coating by means of dip coating, electrospinning and/or micropippetting.

11. Method according to any one of claims 1 - 10, wherein at least one antiproliferative, anti-migration, antiangiogenic, anti-inflammatory, antiphlogistic, cytostatic, cytotoxic and/or antithrombotic active agent is applied and/or incorporated beneath, in and/or on the layer of polymer A and/or beneath, in and/or on the layer of polymer B or portions of said layers.

12. Method according to claim 11, wherein the antiproliferative, anti-migration, anti-angiogenic, anti-inflammatory, antiphlogistic, cytostatic, cytotoxic and/or antithrombotic active agent is selected from the group comprising: sirolimus (rapamycin), everolimus, pimecrolimus, somatostatin, tacrolimus, roxithromycin, daunaimycin, ascomycin, bafilomycin, erythromycin, midecamycin, josamycin, concanamycin, clarithromycin, troleandomycin, folimycin, cerivastatin, simvastatin, lovastatin, fluvastatin, rosuvastatin, atorvastatin, pravastatin, pitavastatin, vinblastine, vincristine, vindesine, vinorelbine, etoposide, teniposide, nimustine, carmustine, lomustine, cyclophosphamide, 4-Hydroxyoxycyclophosphamide, estramustine, melphalan, ifosfamide, trofosfamide, chlorambucil, bendamustine, dacarbazine, busulfan, procarbazin, treosulfan, temozolomide, thiotepa, Doxorubicin, aclarubicin, epirubicin, mitoxantrone, idarubicin, bleomycin, mitomycin, dactinomycin, methotrexate, fludarabine, fludarabine-5'-dihydrogenphosphate, cladribine, mercaptopurine, thioguanine, cytarabine, fluorouracil, gemcitabine, capecitabine, Docetaxel, carboplatin, cisplatin, oxaliplatin, amsacrine, irinotecan, topotecan, hydroxycarbamide, miltefosine, pentostatin, aldesleukin, tretinoin, asparaginase, pegaspargase, anastrozole, exemestane, letrozole, formestane, aminoglutethimide, adriamycin, azithromycin, spiramycin, cepharanthin, 8-α-ergolines, dimethyl ergoline, agroclavin, 1-allyl lisuride, 1-allyl terguride, bromerguride, bromocriptine, elymoclavine, ergocristine, ergocristinine, ergocornine, ergocorninine, ergocryptine, ergocryptinine, ergometrine, ergonovine, ergosine, ergosinine, ergometrinine, ergotamine, ergotaminine, ergovaline, lergotrile, lisuride, lysergol, lysergic acid, lysergic acid amide, lysergic acid diethylamide, isolysergic acid, isolysergic acid amide, isolysergic acid diethylamide, mesulergine, metergoline, methergine, methylergometrine, methysergide, pergolide, proterguride and terguride, celecoxib, thalidomide, Fasudil^{®}, cyclosporine, inhibitor-2ω of SMC proliferation, epothilones A and B, mitoxanthrone, azathioprine, mycophenolate mofetil, c-myc antisense, b-myc antisense, betulinic acid, camptothecin, PI-88 (sulphated oligosaccharide), melanocyte stimulating hormone (α-MSH), activated protein C, IL1-β-Inhibitor, Thymosin α-1, fumaric acid and esters thereof, calcipotriol, tacalcitol, lapachol, β-lapachone, podophyllotoxin, betulin, 2-ethylhydrazide of podophyllic acid, molgramostim (rhuGM-CSF), peginterferon α-2b, lanograstim (r-HuG-CSF), filgrastim, macrogol, dacarbazine, basiliximab, daclizumab, selectin (cytokine antagonist), CETP inhibitor, cadherins, cytokine inhibitors, COX-2-Inhibitor, NFkB, angiopeptin, ciprofloxacin, fluroblastin, monoclonal antibodies inhibiting the muscle cell proliferation, bFGF antagonists, probucol, prostaglandins, 1,11-dimethoxycanthin-6-one, 1-hydroxy-11-methoxycanthin-6-one, scopoletin, colchicine, NO donors such as pentaerythritol tetranitrate and sydnonimines, S-nitroso derivatives, tamoxifen, staurosporine, β-estradiol, α-estradiol, estriol, estrone, ethinyl estradiol, fosfestrol, medroxyprogesterone, estradiol cypionates, estradiol benzoates, tranilast, kamebaukarin and other terpenoids applied in the therapy of cancer, verapamil, tyrosine kinase inhibitors (tyrphostines), cyclosporine A, paclitaxel and derivatives thereof such as 6-α-hydroxy-paclitaxel, baccatine, taxotere, macrocyclic oligomers of carbon suboxide (MCS) obtained synthetically and from native sources and derivatives thereof, mofebutazone, acemetacin, diclofenac, lonazolac, dapsone, o-carbamoyl-phenoxyacetic acid, lidocaine, ketoprofen, mefenamic acid, piroxicam, meloxicam, chloroquine phosphate, penicillamine, tumstatin, avastin, D-24851, SC-58125, hydroxychloroquine, auranofin, sodium aurothiomalate, oxaceprol, celecoxib, β-sitosterin, ademetionine, myrtecaine, polidocanol, nonivamide, levomenthol, benzocaine, aescin, ellipticine, D-24851 (Calbiochem), colcemid, cytochalasin A-E, indanocine, nocadazole, protein S 100, bacitracin, vitronectin receptor antagonists, azelastine, guanidyl cyclase stimulator, tissue inhibitor of metal proteinase 1 and 2, free nucleic acids, nucleic acids incorporated into virus transmitters, DNA and RNA fragments, plasminogen activator inhibitor 1, plasminogen activator inhibitor 2, antisense oligonucleotides, VEGF inhibitors, IGF-1, active agents from the group of antibiotics such as cefadroxil, cefazolin, cefaclor, cefoxitin, tobramycin, gentamycin, penicillins such as dicloxacillin, oxacillin, sulfonamides, metronidazole, antithrombotics such as argatroban, aspirin, Abciximab, synthetic antithrombin, bivalirudin, coumadin, enoxoparin, desulphated and N-reacetylated heparin, tissue plasminogen activator, GpIIb/IIIa platelet membrane receptor, factor Xₐ inhibitor antibody, heparin, hirudin, r-hirudin, PPACK, protamine, sodium salt of 2-methylthiazolidine-2,4-dicarboxylic acid, prourokinase, streptokinase, warfarin, urokinase, vasodilators such as dipyramidole, trapidil®, nitroprussides, PDGF antagonists such as triazolopyrimidine and seramin, ACE inhibitors such as captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators such as p65, NF-kB or Bcl-xL antisense oligonucleotides, halofuginone, nifedipine, Tocopherol, vitamin B1, B2, B6 and B12, folic acid, molsidomine, tea polyphenols, epicatechin gallate, epigallocatechin gallate, Boswellic acids and derivatives thereof, leflunomide, anakinra, etanercept, sulfasalazine, etoposide, dicloxacillin, tetracycline, triamcinolone, mutamycin, procainimide, D24851, SC-58125, retinoic acid, quinidine, disopyrimide, flecainide, propafenone, sotolol, amidorone, natural and synthetically obtained steroids such as bryophyllin A, inotodiol, maquiroside A, ghalakinoside, mansonine, strebloside, hydrocortisone, betamethasone, dexamethasone, non-steroidal substances (NSAIDS) such as fenoprofen, ibuprofen, indomethacin, naproxen, phenylbutazone and other antiviral agents such as acyclovir, ganciclovir and zidovudine, antimycotics such as clotrimazole, flucytosine, griseofulvin, ketoconazole, miconazole, nystatin, terbinafine, antiprotozoal agents such as chloroquine, mefloquine, quinine, moreover natural terpenoids such as hippocaesculin, barringtogenol-C21-angelate, 14-Dehydroagrostistachin, agroskerin, agroskerin, agrostistachin, 17-Hydroxyagrostistachin, ovatodiolids, 4,7-oxycycloanisomelic acid, baccharinoids B1, B2, B3 and B7, tubeimoside, bruceanoles A, B and C, bruceantinoside C, yadanziosides N and P, isodeoxyelephantopin, tomenphantopin A and B, coronarin A, B, C und D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, moreover cymarin, apocymarin, aristolochic acid, aminopterin, hydroxyanopterin, anemonin, protoanemonin, berberine, cheliburin chloride, cicutoxin, sinococuline combrestatin A and B, cudraisoflavone A, curcumin, dihydronitidine, nitidine chloride, 12-β-hydroxypregnadien-3,20-dione, bilobol, ginkgol, ginkgolic acid, helenalin, indicine, indicine-N-oxide, lasiocarpine, glycoside 1α, justicidin A and B, larreatin, malloterin, mallotochromanol, isobutyrylmallotochromanol, maquiroside A, marchantin A, maytansin, lycoridicin, margetine, pancratistatin, liriodenine, oxoushinsunine, aristolactam-All, bisparthenolidine, periplocoside A, ursolic acid, deoxypsorospermin, psycorubin, ricin A, sanguinarine, manwu wheat acid, methylsorbifolin, chromones from spathelia, stizophyllin, mansonine, akagerine, dihydrousambaraensine, hydroxyusambarine, strychnopentamine, strychnophylline, usambarine, usambarensine, daphnoretin, lariciresinol, methoxylariciresinol, syringaresinol, umbelliferone, afromoson, acetylvismione B, desacetylvismione A, vismione A and B and sulphurous amino acids such as cystine as well as salts, hydrates, solvates, enantiomers, racemates, enantiomer mixtures, diastereomeric mixtures and mixtures of the above active agents mentioned above.

13. Method according to any one of claims 1 - 12, wherein the endoprosthesis to be coated has a tubular, helical and/or braided structure.

14. Method according to any one of claims 1 - 12, wherein the endoprosthesis to be coated is a stent, coronary stent, vascular stent, tracheal stent, bronchial stent, urethral stent, esophageal stent, biliary stent, renal stent, stent for use in the small intestine, stent for use in the large intestine, laryngeal implant, bypass, catheter or ileostomy.

15. Endoprosthesis which can be obtained according to any one of claims 1 - 14.

16. Endoprosthesis according to claim 15 suited for the prevention, reduction or treatment of stenosis, restenosis, arteriosclerosis, atherosclerosis, vessel occlusions, vessel constrictions, aneurysms, and for artificial openings and accesses.

## Revendications

1. Un procédé pour le revêtement de toute la surface d'un endoprothèse en forme de grille ou de filet comprenant les étapes suivantes :
a) fournir un endoprothèse en forme de grille ou de filet ayant une surface discontinue avec des interstices entre les entretoises formant la grille ou le filet,
b) au moins revêtir partiellement les entretoises avec un polymère A,
c) mouiller la surface des entretoises revêtues avec le polymère A avec un solvant organique,
d) revêtir toute la surface des interstices se trouvant entre les entretoises formant la grille ou le filet avec un revêtement polymérique d'un polymère B, où le revêtement de la surface totale avec le polymère B est plus longue que la longuer de l'endoprothèse et dans une étape supplémentaire est placé sur les bords de l'endoprothèse vers l'extérieur et les bords résultants sont intégrés sous pression et à température élevée dans la couche sous-jacente du polymère B.

2. Le procédé pour le revêtement de toute la surface de l'endoprothèse en forme de grille ou de filet selon la revendication 1 comprenant les étapes suivantes:
a) fournir un endoprothèse en forme de grille ou de filet ayant une surface discontinue comprenant des entretoises avec une surface intérieure et une surface extérieure avec des interstices entre les entretoises individuelles,
b) au moins revêtir partiellement la surface intérieure et la surface extérieure des entretoises avec un polymère A,
c) mouiller la surface de l'endoprothèse revêtue avec le polymère A avec un solvant organique,
d) revêtir complètement la surface intérieure et / ou extérieure, ainsi que les interstices entre les entretoises avec un revêtement polymérique d'un polymère B, où le revêtement de la surface totale avec le polymère B est plus longue que la longuer de l'endoprothèse et dans une étape supplémentaire est placé sur les bords de l'endoprothèse vers l'extérieur et les bords résultants sont intégrés sous pression et à température élevée dans la couche sous-jacente du polymère B.

3. Le procédé selon la revendication 1 ou 2, où le polymère A et le polymère B sont identiques.

4. Le procédé selon la revendication 1 ou 2, dans lequel l'étape de revêtement b) est effectuée par pulvérisation ou par filage électrostatique.

5. Le procédé selon la revendication 1 ou 2, dans lequel les interstices entre les entretoises ne sont pas revêtus avec une couche polymérique à l'étape de revêtement b).

6. Le procédé selon la revendication 1 ou 2, dans lequel le mouillage selon l'étape c) est réalisée par trempage ou pulvérisation.

7. Le procédé selon une des revendications 1 - 6, dans lequel le solvant organique utilisé pour le mouillage selon l'étape c) contient le polymère B dans une concentration qui est inférieure à la concentration du polymère B dans la solution selon l'étape d).

8. Le procédé selon une des revendications 1 - 7, dans lequel le polymère A et/ou le polymère B est choisi dans le groupe comprenant de l'acide polyacrylique et des polyacrylates tels que poly-méthacrylate de méthyle, poly-méthacrylate de butyle, polyacrylamide, polyacrylonitriles, polyamides, polyétheramides, polyéthylène amine, polyimides, polycarbonates, polycarbouréthanes, polyvinylcétones, polyvinyle halogénures, polyvinylidène halogénures, éthers polyvinyliques, aromatiques polyvinyliques, esters polyvinyliques, pyrrolidones polyvinyliques, polyoxyméthylènes, polyéthylène, polypropylène, polytétrafluoroéthylène, polyuréthanes, polyoléfine élastomères, polyisobutylènes, gommes EPDM, fluorosilicones, carboxyméthyl chitosans, polyéthylène téréphthalate, polyvalérates, carboxyméthylcellulose, cellulose, rayonne, rayonne triacétates, cellulose nitrates, cellulose acétates, hydroxyéthyl cellulose, cellulose butyrates, cellulose acétate butyrates, éthyle vinyle acétate copolymères, polysulfones, polyéthersulfones, résines époxy, résines ABS, gommes EPDM, silicones comme polysiloxanes, polydiméthylsiloxanes, polyvinyl-halogénes et copolymères, cellulose éthers, cellulose triacétates, chitosane, dérivés du chitosane, des huiles polymérisables comme par exemple l'huile de lin et copolymères et/ou mélanges des ceux-ci.

9. Le procédé selon une des revendications 1 - 7, dans lequel le polymère A et/ou le polymère B est choisi dans le groupe comprenant polyvalérolactones, poly-ε-décalactone, polylactides, polyglycolides, copolymères polylactides/polyglycolides, poly-ε-caprolactone, polyhydroxybutyrates, polyhydroxyvalérates, polyhydroxybutyrate-co-valérates, poly(1,4-dioxane-2,3-diones), poly(1,3-dioxane-2-ones), poly-para-dioxanones, polyanhydrides comme poly(anhydride maléique), poly-hydroxy-méthacrylates, fibrine, poly-cyano-acrylates, polycaprolactone diméthylacrylates, acide poly-β-maléique, polycaprolacton-butyl-acrylates, polymères à blocs multiples comme par example d'oligocaprolactondiols et d'oligodioxanondiols, polyéther-ester-polymères à blocs multiples comme par exemple de PEG et de polybutylène téréphthalate, polypivotolactones, acide polyglycolique triméthyl carbonates, polycaprolactone glycolides, poly(γ-éthyl-glutamate), poly(DTH-iminocarbonate), poly(DTE-co-DT-carbonate), poly(bisphénol A-iminocarbonate), polyorthoesters, acide polyglycolique triméthyl carbonates, polytriméthyl carbonates, polyiminocarbonates, poly(N-vinyl)-pyrrolidone, polyvinyl alcohols, polyester amides, polyesters glycolés, polyphosphoesters, polyphosphazènes, poly[p-carboxyphénoxy)propane], acide polyhydroxy-péntanoique, polyanhydrides, polyéthylène oxyde propylène oxyde, polyuréthanes mous, polyuréthanes ayant des résidus amino acide dans le squelette, polyétheresters comme polyéthylène oxyde, polyalkènoxalates, polyorthoesters ainsi que copolymères de ceux-ci, carraghénane, fibrinogène, starch, collagène, polymères à base de protéine, poly(amino acides), poly(amino acides) synthétiques, zéine, zéine modifiée, polyhydroxyalkanoates, acide pectique, acide actinique, fibrine et caséine modifiée et non-modifiée, albumine, en outre acide hyaluronique, héparansulfate, héparine, chondroïtine sulfate, dextrane, β-cyclodextrines, et copolymères avec PEG et polypropylène glycol, gomme arabique, guar, gélatine, collagène, collagène N-hydroxysuccinimide, des modifications et copolymères et/ou mélanges des substances mentionnées ci-dessus.

10. Le procédé selon une des revendications 1 - 9, dans lequel l'endoprothèse est revêtu par trempage, électrofilage et / ou micropipetage avec un revêtement de polymère sur toute la surface.

11. Le procédé selon une des revendications 1 - 10, dans lequel au moins un agent anti-prolifératif, antimigrative, anti-angiogénique, anti-inflammatoire, antiphlogistique, cytostatique, cytotoxique et / ou antithrombogénique est appliqué et/ou incorporé en-dessous, dans et/ou au-dessus de la couche de polymère A et/ou en-dessous, dans et/ou au-dessus de la couche de polymère B ou des portions de celles-ci.

12. Le procédé selon la revendication 11, dans lequel l'agent anti-prolifératif, antimigrative, anti-angiogénique, anti-inflammatoire, antiphlogistique, cytostatique, cytotoxique et / ou antithrombogénique est choisi à partir du groupe comprenant : sirolimus (rapamycine), everolimus, pimecrolimus, somatostatine, tacrolimus, roxythromycine, dunaimycine, ascomycine, bafilomycine, érythromycine, midécamycine, josamycine, concanamycine, clarithromycine, troleadomycine, folimycine, cérivastatine, simvastatine, lovastatin, fluvastatin, rosuvastatine, atorvastatine, pravastatine, pitavastatine, vinblastine, vincristine, vindesine, vinolrebine, étoposide, teniposide, nimustine, carmustine, lomustine, cyclophosphamide, 4-hydroxyoxycyclo phosphamide, éstramustine, melphalan, ifosfamide, trofosfamide, chlorambucil, bendamustine, dacarbazine, busulfan, procarbazine, tréosulfan, trémozolomide, thiotépa, daunorubicine, doxorubicine, aclarubicine, épirubicine, mitoxantrone, idarubicine, bléomycine, mitomycine, dactinomycine, méthotrexate, fludarabine, fludarabine-5'-dihydrogéne phosphate, cladribine, mércaptopurine, thioguanine, cytarabine, fluorouracile, gemcitabine', capécitabine, docétaxel, carboplatine, cisplatine, oxaliplatin, amsacrine, irinotécan, topotécan, hydroxycarbamide, miltéfosine, pentostatine, aldesleukine, trétinoine, asparaginase, pegaspargase, anastrozole, exémestane, létrozole, formestane, aminoglutéthimide, adriamycine, azithromycine, spiramycine, cépharanthine, 8-α-ergoline, diméthylergoline, agroclavine, 1-allyl lisuride, 1-allyl terguride, bromerguride, bromocriptine, elymoclavine, érgocristine, érgocristinine, érgocornine, érgocorninine, érgocryptine, érgocryptinine, érgométrine, érgonovine, érgosine, érgosinine, érgométrinine, érgotamine, érgotaminine, érgovaline, lergotril, lisuride, lysergol, acide lysergique, amide de l'acide lysergique, diéthylamide de l'acide lysergique, acide isolysergique, amide de l'acide isolysergique, diéthylamide de l'acide isolysergique, mesulergin, métergoline, méthergine, méthylergométrine, pergolide, proterguride et terguride, celecoxip, thalidomide, Fasudil^{®}, ciclosporine, inhibiteur de la prolifération de cellules des muscles lisses-2w, épothilone A et B, mitoxanthrone, azathioprine, mycophénolate mofétil, b-myc antisense, c-myc antisense, acide bétulinique, camptothécine, PI-88 (oligosaccharide sulfaté), hormone mélanotrope (α-MSH), protéine C activée, inhibiteur de l'IL1-β, thymosine α-1, acide fumarique et ses esters, calcipotriol, tacalcitol, lapachol, β-lapachone, podophyllotoxine, bétuline, acide podophyllique-2-éthylhydrazide, molgramostim (rhuGM-CSF), pegintérferon α-2b, lenograstim (r-HuG-CSF), filgrastim, macrogol, dacarbazin, basiliximab, daclizumab, sélectine (cytokine antagoniste), inhibiteur de la CETP, cadhérine, inhibiteurs des cytokines, inhibiteur de la COX-2, NFkB, angiopeptine, ciprofloxacine, camptothécine, fluroblastin, anticorps monoclonaux qui inhibitent la prolifération des cellules musculaires, antagonistes du bFGF, probucol, prostaglandines, 1, 11-diméthoxycanthin-6-one, 1-hydroxy-11-méthoxycanthin-6-one, scopolétol, colchicine, donneurs de NO comme le pentaérythrityl tetranitrate et les sydnoimines, dérivés S-nitroso, tamoxifène, staurosporine, β-estradiol, α-estradiol, estriol, estrone, éthinyl estradiol, fosfestrol, médroxyprogestérone, cypionates d'estradiol, benzoates d'estradiol, tranilast, kamebakaurine et autres terpénoides utilisés dans la thérapie du cancer, verapamil, inhibiteurs de la tyrosine kinase (tyrphostins), ciclosporine A, paclitaxel et ses dérivés comme 6-α-hydroxy-paclitaxel, baccatin, taxotères, oligomères macrocycliques du suboxyde de carbone obtenus synthétiquement et à partir des sources naturelles et leurs dérivés, mofebutazone, acemétacine, diclofenac, lonazolac, dapsone, acide o-carbamoyl-phenoxyacétique, lidocaine, kétoprofène, acide méfénamique, piroxicam, méloxicam, chloroquine phosphate, pénicillamine, tumstatine, avastine, D-24851, SC-58125, hydroxychloroquine, auranofine, aurothiomalate de sodium, oxacéprol, célécoxib, β-sitostérol, ademétionine, myrtécaïne, polidocanol, nonivamide, lévomenthol, benzocaine, aescin, ellipticine, D-24851 (Calbiochem), colcémid, cytochalasines A-E, indanocine, nocodazole, protéine S100, bacitracine, antagonistes du récepteur de la vitronectine, azélastine, stimulateur de la guanidyl cyclase, inhibiteur tissulaire des métalloprotéinases 1 et 2, acides nucléiques libres, acides nucléiques incorporés dans des virus transmetteurs, fragments de l'ADN et l'ARN, inhibiteur-1 de l'activateur du plasminogène, inhibiteur-2 de l'activateur du plasminogène, les oligonucléotides antisens, les inhibiteurs du VEGF, l'IGF-1, agents actifs du groupe des antibiotiques comme céfadroxil, céfazolin, céfaclor, céfoxitin, tobramycine, gentamicine, pénicillines comme dicloxacilline, oxacilline, sulfonamide, métronidazole, antithrombotiques comme argatroban, aspirine, abciximab, antithrombine synthétique, bivalirudine, coumadin, énoxaparin, héparines desulfatées et N-ré-acétylés, activateur tissulaire du plasminogène, récepteur membranaire de la plaquette GpIIb/IIIa, anticorps de l'inhibiteur du facteur Xa, héparine, hirudine, r-hirudine, PPACK, protamine, sel de sodium de l'acide 2-méthylthiazolidin-2,4-dicarboxylique, prourokinase, streptokinase, warfarine, urokinase, vasodilatateurs comme dipyramidole, Trapidil®, nitroprusside, antagonistes du PDGF comme triazolopyrimidine et seramin, inhibiteurs de l'ACE comme captopril, cilazapril, lisinopril, énalapril, losartan, inhibiteurs de la thioprotéase, prostacycline, vapiprost, interféron α, β et γ, antagonistes d'histamine, bloqueurs de la sérotonine, inhibiteurs de l'apoptose, régulateurs de l'apoptose comme p65, NF-kB ou des oligonucléotides antisense Bcl-xL, halofuginone, nifédipine, tocophérol, vitamine B1, B2, B6 et B12, acide folique, tranilast, molsidomine, polyphénols du thé, gallate d'épicatéchine, gallate d'épigallocatéchine, acides boswelliques et leurs dérivés, léflunomide, anakinra, étanercept, sulfasalazine, etoposid, dicloxacylline, tétracycline, triamcinolone, mutamycine, procainimide, D24851, SC-58125, acide rétinoïque, quinidine, disopyrimide, flécaïnide, propafénone, sotolol, amidorone, stéroïdes naturels et synthétiques comme bryophylline A, inotodiol, maquiroside A, ghalakinoside, mansonine, strebloside, hydrocortisone, bétaméthasone, déxaméthasone, substances non-stéroïdiennes (NSAIDS) comme fénoprofène, ibuprofène, indomèthacine, naproxen, phénylbutazone et d'autres agents antiviraux comme acyclovir, ganciclovir et zidovudine, antimycotiques comme clotrimazole, flucytosine, griséofulvine, kétoconazole, miconazole, nystatin, terbinafine, agents antiprotozoaires comme chloroquine, méfloquine, quinine, d'autres terpénoïdes naturels comme hippocaesculine, barringtogénol-C21-angelate, 14-déhydroagrostistachine, agroskerine, agrostistachine, 17-hydroxyagrostistachine, ovatodiolids, acide 4,7-oxycycloanisomelique, baccharinoids B1, B2, B3 et B7, tubeimoside, bruceantinoside C, yadanziosides N et P, isodéoxyelephantopine, tomenphantopine A et B, coronarine A, B, C et D, acide ursolique, acide hyptatique A, zeorin, iso-iridogermanal, maytenfoliol, effusantine A, excisanine A et B, longikaurine B, sculponeatin C, kamebaunin, leukamenin A et B, 13,18-déhydro-6-alpha-senecioyloxychaparrin, taxamairin A et B, regenilol, triptolide, en outre cymarin, apocymarine, acide aristolochique, anopterine, hydroxyanopterine, anémonin, protoanémonin, berbérine, chlorure de cheliburin, cictoxine, sinococuline, bombrestatin A et B, cudraisoflavone A, curcumine, dihydronitidine, chlorure de nitidine, 12-β-hydroxypregnadièn-3,20-dione, bilobol, ginkgol, acide ginkgolique, hélénaline, indicine, indicine-N-oxyde, lasiocarpine, inotodiol, glycoside 1a, podophyllotoxin, justicidine A et B, larréatin, malloterin, mallotochromanol, isobutyrylmallotochromanol, maquiroside A, marchantine A, maytansine, lycoridicine, margétine, pancratistatin, liriodènine, oxoushinsunine, aristolactame-All, bisparthenolidine, periplocoside A, ghalakinoside, acide ursolique, déoxypsorospermine, psychorubine, ricin A, sanguinarine, acide du blé manwu, méthylsorbifoline, chromones de spathelia, stizophylline, mansonine, streblosid, akagerine, dihydro usambaraensine, hydroxyusambarine, strychnopentamine, strychnophylline, usambarine, usambarensine, berbérine, liriodenine, oxoushinsunine, daphnoretine, laricirésinol, méthoxylaricirésinol, syringarésinol, umbellifèrone, afromosone, acétylvismione B, desacétylvismione A, vismione A et B, et des acides aminés contenant du souffre comme cystine, ainsi que des sels, hydrates, solvates, énantiomères, racémates, mélanges d'énantiomères, mélanges de diastéréoisomères et mélanges des substances mentionnées ci-dessus.

13. Le procédé selon une des revendications 1 - 12, dans lequel l'endoprothèse à revêtir présente une structure tubulaire, en spirale, et/ou tressée.

14. Le procédé selon une des revendications 1 - 12, dans lequel l'endoprothèse à revêtir est un stent, un stent coronaire, un stent vasculaire, un stent trachéen, un stent bronchique, un stent urétral, un stent oesophagien, un stent biliaire, un stent pour les reins, un stent pour l'intestin grêle, un stent pour le colon, un implant laryngien, un bypass, un cathéter ou un anus artificiel.

15. Un endoprothèse obtenable par le procédé selon une des revendications 1 - 14.

16. L'endoprothèse selon la revendication 15 pour la prévention, la réduction ou le traitement de la sténose, la resténose, l'athérosclérose, l'athérosclérose, des occlusions vasculaires, des rétrécissements vasculaires, des anévrismes, des sorties et des ajouts artificiels.
